# EUROPEAN PATENT SPECIFICATION

(11) **EP 1 786 338 B1**
(45) Date of publication and mention of the grant of the patent: **12.02.2020**
(21) Application number: 05771803.3
(22) Date of filing: 13.08.2005
(51) Int. Cl.: A61B 17/28, A61B 17/02

(54) **DISTRACTION AND RETRACTION ASSEMBLIES**
DISTRAKTIONS- UND RETRAKTIONSANORDNUNGEN
ENSEMBLES POUR DISTRACTION ET RÉTRACTION

(30) Priority: 15.08.2004 AU 2004904580
(43) Date of publication of application: 23.05.2007
(73) Proprietor: Seex, Kevin, Kingswood, NSW 2747 (AU)
(72) Inventor: Seex, Kevin, Kingswood, NSW 2747 (AU)
(74) Representative: Tollett, Ian
(86) International application number: PCT/AU2005/001205
(87) International publication number: WO 2006/017886

(56) References cited:
- WO-A1-98/35622
- WO-A2-2004/030546
- CA-A1- 2 508 789
- DE-A1- 4 300 307
- DE-A1- 4 440 035
- GB-A- 2 198 647
- RU-C2- 2 230 516
- SU-A1- 578 957
- SU-A1- 660 671
- SU-A1- 1 503 767
- US-A- 4 462 403
- US-A- 5 575 805
- US-A1- 2002 123 754
- US-A1- 2004 024 411
- US-B1- 6 214 028

## Description

### BACKGROUND

The present invention relates to distraction and retraction assemblies. The invention also provides an assembly which performs the functions of distraction and retraction optimising mechanical advantage and efficiency in retraction and resisting unwanted pull out of retractors.

### PRIOR ART

There are in existence a number of assemblies used in retraction of tissues to facilitate spinal and particularly cervical surgery.

The most commonly performed anterior cervical procedure is an intervertebral fusion procedure that typically involve the steps of removing a portion or all of the affected disc material, spreading apart adjacent vertebrae with a distractor, and inserting an implant bone or cage or prosthetic disc into the space previously occupied by the removed disc material. This procedure can be done either from the front of the patient (anterior interbody fusion) or in the lumbar spine from the back (posterior interbody fusion). If done from the front, it is important to reduce the size of the retraction forces applied by the blades of the of the retractor so that the procedure is as minimally invasive as possible and thus minimally interferes with and minimally traumatizes the organs, tissues and vasculature being displaced to allow access to the vertebral region being treated. Posterior surgery can utilize larger tools since the insertion space is more accommodating and posterior structures requiring retraction i.e. muscles are less sensitive.

By way of an example of a known device, United States Patent 6,669,699 discloses a Distraction instrument for use in anterior cervical fixation surgery. An intervertebral distraction tool has a clamshell head with upper and lower halves, each having a curved outer surface and a flat inner surface. The distal side of the head is hinged so that the head opens and closes from the proximal side of the head. The hinge is a separating hinge that allows the halves to not only angulate with respect to one another about the hinge axis, but also to vertically separate from one another at the hinge. The distraction separator has decreasing taper at its distal end and a longitudinal bore that accommodates the elongate shaft of the trial so that the separator can be moved longitudinally relative to the shaft. Upon forward movement of the separator, the tapered upper and lower surfaces engage the flat inner surfaces of the head, causing the halves to angulate about the hinge axis of the [head, thereby opening the head.

In another example United States Patent 6,743,231 discloses temporary spinal fixation apparatuses and methods for temporarily fixing the relative position of spinal implant assemblies until a permanent fixation position is determined. The disclosed apparatuses and methods enhance the ease of placement of spinal implant assemblies and facilitates the accuracy of positioning of the spinal vertebrae. This apparatus can reduce the number of steps needed to perform spinal surgery and can decrease the likelihood of post-operative complications.

In another example United States Patent 6,017,342 discloses a compression and distraction instrument having two pivotally connected handles. Jaw portions engage objects, such as human bone, for purposes of manouvering. A control screw connects with the handles, through a mechanical advantage arrangement, and the screw pivots the jaw portions for the engagement of the objects. There is an anti-friction connection between the screw and the handles, for accurate and precise movement of the jaws. In one example, the screw is axial of the instrument, and, in the other example, the screw is transverse thereto, both have mechanical advantage.

In another example United States Patent 6,712,825 of March 30, 2004 discloses a spinal disc space distracter for separating adjacent elements, such as vertebrae. The distracter preferably has a scissors-type distracting mechanism, either in a simple scissors or double-acting scissors configuration. The distracter includes blades that are removable from the jaws of the distracter such that different blades may be used depending on the patient and situation with which the distracter is to be used. The jaws include a mating fixture and the blades include a mating portion for removable association with the mating fixture. In accordance with the principles disclosed, a spinal disc distracter is provided to allow for an implant insertion technique to be performed during distraction of the disc space. The implants are slid into the disc space between the distracter blades, preferably while the blades are in contact with the upper and lower surfaces of the adjacent vertebral bodies. The distracter is formed to be as minimally invasive and atraumatic as possible such that it may readily be used in an anterior or anterolateral approach.

Thus, the distracter is configured to be used in the confined spaces of the human anatomy through a small surgical incision. This distracter has a scissor-like configuration with a pair of handles pivotally connected together. A distracter jaw is coupled to a distal end of each handle such that movement of the handles together draws the jaws apart to separate the vertebrae being treated. The jaws and handles are pivotally coupled together in a double-acting scissor-like configuration to further reduce the space required to move the jaws apart and thus further minimize the invasiveness of the device and procedure. Although the handles, jaws, and distracter mechanism may all lie in the same plane, in order to facilitate visualization of the treatment site during distraction and insertion of an implant, at least the handles may be angled away from the plane of the distracter jaws.

A locking mechanism preferably is provided adjacent to or in the handle to maintain distraction. The locking mechanism may include a spindle or threaded bolt mounted on a first handle and passing through the second handle. An internally threaded speed nut is rotatably mounted on the threaded bolt such that movement of the speed nut along the bolt selectively inhibits movement of the second handle away [from the first handle and thus maintains the vertebrae at the desired distracted position].

The blades of the distracter are configured to increase versatility of the distracter and are removably coupled to the distracter jaws. Thus, the blades may be changed, as necessary or desired, for a given procedure or patient.

In another example, the blades of the distracter are gradually curved to be out of the plane of the distracter mechanism. Because of the gradual curve, the distal end of the jaws may safely be manipulated through the patient's body with as minimal contact as possible with organs and vasculature including major blood vessels. Moreover, such curvature permits insertion through a smaller incision because of the increased manipulability of the gradually curved blades through small openings and spaces.

In another example of a distracter assembly United States Patent 6,740,119 May 25, 2004 discloses a method of distracting vertebral bones to their proper anatomical spacing including sequentially inserting and removing a series of progressively wider cylindrical spacer elements into the intervertebral space between adjacent vertebral bones until the distance between the vertebral bones is anatomically appropriate

There are limitations inherent in conventional self retaining retractors used widely in surgery and routinely in anterior spinal surgery. These typically have two blades typically with teeth at one end that are linked by a hinge to scissor type handles. The teeth grip into or under the tissues and apply lateral forces as the handles are compressed by the surgeon to produce the desired exposure. The teeth grip and retract both sides of a wound against each other with equal distribution of retraction force. This means that when retracting two sides with different resistances eg left and right sides of an anterior (front) cervical wound (trachea, larynx ,ET tube ,oesophagus and thyroid are on one side only) excessive forces and retraction are applied to the easy side in an effort to retract the opposite more resistant side. This is one reason why conventional retractors slip, twist and rise up. Placing the teeth of these retractors under the longus colli muscle (next to the spine) works to an extent and is the standard method by which retractors in the anterior (front) of the neck are secured but it is common for retractors to require repositioning several times during an operation in addition to the difficulty of securing them the correct place initially. Teeth cannot be made too sharp or too long as they will damage vital structures. The second reason they slip is because the retraction forces are coming from the top ie outside the wound at a distance to where they at required at the bottom of the wound. The path of least resistance is up and as the retractor opens even the slight bending tends to produce a vector of force up as the blade follows the path of least resistance out of the wound leading to unwanted displacement of the retractor and therefore compromised retraction. The third reason they slip is that the tissues under retraction stretch reducing their counter force. As self retaining retractors rely on counter force for stability as this is reduced loosening inevitably occurs.

Repeated adjustment can and does produce unnecessary tissue injury e.g. swallowing problems or hoarseness of voice in perhaps 5 - 10% of anterior cervical operations. It also wastes time and produces unnecessary bleeding. Persons skilled in the art are aware that one of the most frustrating parts of this type of surgery is positioning and maintaining position of the retractors. Conventional retractors are sometimes weighed down with chains and weights to resist the unwanted upwards rotational forces.

In another example of the prior art GB 2 198 647 A (JUNIPER) discloses a scissor type distractor which includes a retractor am. It discloses an assembly capable of retraction of soft tissue and distraction during surgery on a a temporomandibular joint (but not spinal surgery). Juniper does not show a distal end pin which engages vertebral bone and which anchors and co -operates with a first member, nor a distal end pin which engages vertebral bone and anchors a second member. The device does not allow retraction to accommodate distraction movements during distraction. In Juniper the retraction is set then distraction is effected independently of the retractor and there is no transmission of distraction load from the distal end of the scissor to a retractor support allowing the retractor support to move in unison with distraction pins.

Another scissor action surgical tool is disclosed in WO9835622 (A1). This documents teaches gripping forceps having a bifurcated gripping nose on one end adapted to pivotally engage an implant while a rod passes between the branches of the bifurcated gripping nose. The forceps further define a fulcrum adjacent the griping nose. The pivotally engaging gripping nose and included fulcrum permit a surgeon to urge the rod into an opening in an implant with a single instrument while securely gripping the implant. With the implant pivotally engaged by the gripping nose, movement of the forceps towards the rod brings the fulcrum into contact with the rod and further movement urges the rod into an opening in the implant. This device is not described for use as a combined distraction and retraction device.

Some known retractor blades have short spikes for bony fixation that create a point of leverage allowing limited movement of the blade. Other retractor systems allow for fixing a retractor blade to bone within a wound via pins or screws. Some known retractor systems allow rotation of a retractor blade around an axis of rotation attached to a frame that is outside the wound e.g. Synframe™ from Synthes™.

Hohmans™ and Taylor™ retractors have an integral point that is either hammered into bone or pushed into a position to provide bone fixation leveerage. These are widely used in orthopaedic surgery. A Taylor™ type retractor blade is known which incorporates a tube for a securing pin. Also known are lever type retractors such as a hip retractor system designed by Dr R Barry Sorrells. Also know is a rail system for retractor blades and a blade that has means for fixation to bone with screws. Also known is a hip retractor system designed by a Dr S David Stulberg using only pins that are either drilled or hammered into the bone. Some of these can bend or lever slightly but allow no true rotation. External frames for securing levered retractors are also known. For example an assistant (surgeon) free self-retaining hip surgery retractor designed by Dr S David Stulberg is known.

If hammered into bone via a short point fixation, then multiple rotations produces loosening and unwanted withdrawal. Where the pins rest on their point as a simple point of leverage but without deep fixation then they are prone to slippage if knocked or an assistant surgeon is not concentrating. Accordingly, a major disadvantage of the prior art is that there are no retraction systems in use that allow free rotation without compromising fixation.

An anterior cervical system is known whereby retraction blades for cranial and caudal (top and bottom) part of wound are slid over known distraction pins. Additional lateral (side to side) retractors are still required and unconnected.. These blades are loose and can rotate only about axis of pin, (which is not very useful) and are not adjustable. They provide no lateral retraction parallel to spine which is a much greater problem.

The Prodisc™ anterior cervical system for cervical disc replacement has distraction screws with distraction arms that slide down over screws. The Prodisc™ uses a screw cap at top that secures the distraction arm, with downward pressure on to the base of the screw.

### INVENTION

The present invention in one form provides improvements in distraction and retraction assemblies. The invention provides an assembly which performs the functions of distraction and retraction optimising mechanical advantage and efficiency in retraction and resisting unwanted pull out of retractors. More particularly, the invention provides an assembly allowing secure anchorage of retractors and also longitudinal and rotational adjustment of the retractors to adjust retraction forces.

The invention further relates to an assembly which performs the aforesaid retraction functions in conjunction with applied distraction of vertebrae using distraction pins and co operating sleeves so that mechanical advantage in both distraction and retraction is achieved optimising efficiency in retraction and resisting unwanted pull out of retractors.

Although the invention will be described with reference to its surgical applications it will be recognised by persons skilled in the art that the invention has wider applications in retraction alone and in combination retractions and distraction.

During a surgical operation retractors are used to facilitate access to tissues. The present invention employs in one form the principle of fixation into bone ( either directly or indirectly) to provide a secure anchorage and base for a retractor blade and also a stable axis for rotation of the blade within the wound without the prior art unwanted dislocations .

The invention improves tissue exposure and surgical site access and minimizes soft tissue injury, bruising etc (due to the more controlled and reduced movements against tissues) whilst allowing variable selective rotation of at least one retractor blade as required during the surgical procedure. The combined features of the ability to adjust retraction pressure and reduce tissue pressure applied by the retractors thus minimizing tissue injury while maintaining stability of the blade distinguishes the present invention from the known prior art. The direct or indirect fixation (anchorage) to bone of a retractor prevents unwanted slippage and avoids the need for readjustment apart from the selected amount of rotation. The stable axis of rotation from within a surgical wound also imparts a mechanical advantage to retraction pressure reducing operator fatigue. Bone fixation with rotation is achieved in a number of different ways allowing application in numerous anatomical situations.

In each of the embodiments to be described below including the direct fixation retractor assembly and the indirectly anchored retractor and distraction assemblies there exist the following common features:
1 A means for fixation to bone
2. A connection between the point of fixation and retractor (a link)
3. A means for retraction of tissues (a blade)
4. A mechanism for variable rotation dictated by direct or indirect fixation of the blade to bone.

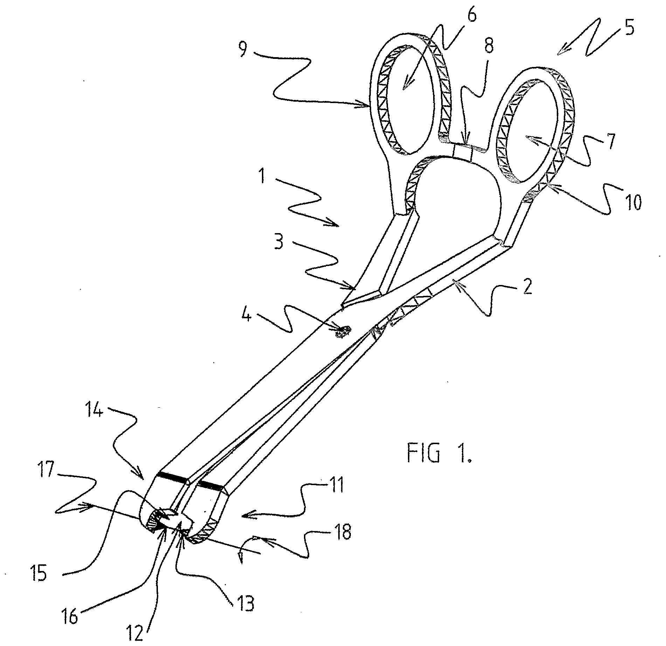

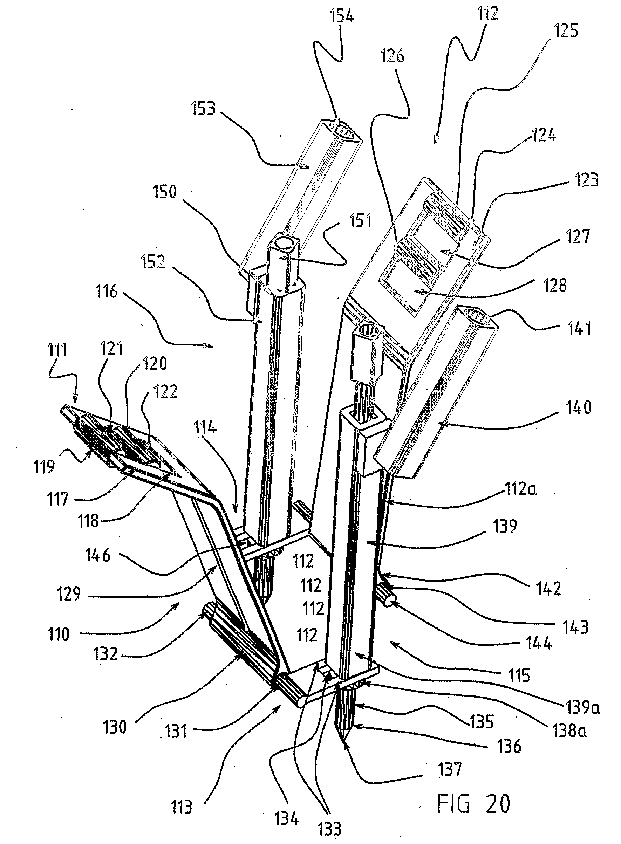

The fixation to bone, link, blade and means of rotation may be direct retractor engagement such as that described with reference to figure 1 below or the fixation may be indirect such as that described with reference to figure 20 accomplished by various combinations of components.

It is, another object of the present invention to provide an improved tissue retraction assembly for retracting wound margins and which provides optimal anchorage of side arms, load application to tissue, rotational and longitudinal adjustment and which may be used in conjunction with vertebral distraction pins.

It is further an object of the present invention to provide an assembly that efficiently and simply manages the insertion of a distractor and retractor. Other objects of the present invention not explicitly stated will be set forth and will be more clearly understood in conjunction with the descriptions of the preferred embodiments disclosed hereafter. The present invention provides an assembly capable of retraction of soft tissue and distraction of bone during spinal surgery as defined in independent claims 1 and 20.

Preferred embodiments are defined in the appended dependent claims.

According to a preferred embodiment each said blades include openings which receive and retain a tool adapted for adjustment of the orientation of said blades.

### BRIEF DESCRIPTION OF THE DRAWINGS

Figure 1 shows a perspective view of a retractor assembly not in accordance with the invention.
Figure 2 shows an elevation view of an alternative retractor assembly, not in accordance with the invention.
Figure 3 shows an enlarged view of a jaw pin of the retractor assembly of figure 2 according to one embodiment
Figure 4 shows a side elevation view of the jaw pin of figure 3.
Figure 5 shows an elevation view of a retractor assembly according to an alternative example, not in accordance with the invention.
Figure 6 shows an enlarged view of a jaw pin and shoulder of the retractor assembly of figure 5.
Figure 7 shows a side elevation view of the jaw pin of figure 6.
Figure 8 shows an elevation view of a retractor assembly according to an alternative example, not in accordance with the invention.
Figure 9 shows an enlarged view of a jaw of the retractor assembly of figure 8.
Figure 10 shows a side elevation view of the enlarged jaw of the formation of figure 9.
Figure 11 shows a perspective view of a retractor assembly **conical anchor** according to one example, not in accordance with the invention.
Figure 12 shows an elevation view of the anchor of figure 11 .
Figure 13 shows an end elevation of the anchor of figure 11.
Figure 14 shows a perspective view of a retractor assembly cup anchor according to an alternative example, not in accordance with the invention.
Figure 15 shows an elevation view of the anchor of figure 14 .
Figure 16 shows an end elevation of the anchor of figure 14.
Figure 17 shows a perspective view of a retractor assembly mushroom anchor according to an alternative example, not in accordance with the invention.
Figure 18 shows an elevation view of the anchor of figure 17 .
Figure 19 shows an end elevation of the anchor of figure 17.
Figure 20 shows a perspective view of a retractor assembly capable of concurrent distraction by co operating anchor pins according to a preferred embodiment of the invention.
Figure 21 shows a perspective view of a retractor assembly retraction arm anchored to an anchor pin by a joining member.
Figure 22 shows an enlarged view of the retractor arm anchor pin of figure 21.
Figure 23 shows an elevation view of the arm anchor pin of figure 22.
Figure 24 shows an elevation view of the arm anchor pin of figure 22.
Figure 25 shows a retractor arm according to an alternative embodiment in a substantially vertical orientation.
Figure 26 shows the retractor arm of figure 25 rotated clockwise from the vertical.
Figure 27 shows a retractor arm and anchorage pin assembly according to an alternative embodiment with the retractor arm in a substantially vertical orientation.
Figure 28 shows the retractor arm of figure 27 rotated clockwise from the vertical.
Figure 29 shows the anchorage pin of figures 27 and 28 with retractor arm released from the anchor head.
Figure 30 shows the retractor arm of figure 31 separated from the joining member
Figure 31 shows a retractor arm and joining member assembly according to an alternative embodiment.
Figure 32 shows an enlarged view of the joining member of figure 31.
Figure 33 shows a retractor arm and joining member assembly according to an alternative embodiment.
Figure 34 shows an enlarged view of the joining member of figure 33.
Figure 35 shows a retractor arm and joining member assembly according to an alternative embodiment.
Figure 36 shows an enlarged view of the joining member of figure 35.
Figure 37 shows an exploded view of a retractor arm and anchorage pin assembly according to an alternative embodiment.
Figure 38 shows a perspective view of the anchor pin shown in figure 37
Figure 39 shows an end elevation of the anchor pin of figure 38.
Figure 40 shows a perspective view of an assembly including an abbreviated retractor arm and joining member including a locating spline assembly according to one embodiment
Figure 41 shows the arrangement of figure 40 with retractor arm rotated to an alternative position disposed normally to the plane of the joining member.
Figure 42 shows an exploded view of the assembly of figure 40.
Figure 43 shows an exploded view of a retraction arm and anchorage assembly according to an alternative embodiment.
Figure 44 shows an assembled view of the retraction arm and anchorage assembly of figure 43.
Figure 45 shows the retractor arm in isolation.
Figure 46 shows a perspective view of anchor showing an oblique recess.
Figure 47 shows a perspective view of a retractor assembly, not in accordance with the invention, of the type described in figure 1 engaging an anchor.

### DETAILED DESCRIPTION

The present invention will now be described in more detail according to a preferred embodiment but non limiting embodiment and with reference to the accompanying illustrations.

Typically according to one exemplary method, cervical distraction of vertebrae involves the use of pins temporarily fixed to the vertebrae to be distracted. Generally two pins are used one above and one below a disc or vertebral body of interest. Traditionally in a Caspar system these pins have only been used for distraction purposes via sliding tubes that fit axially over the pins and connect to an associated distraction mechanism. According to one aspect the role of the pin has been expanded to perform one or more of the following roles. The pin acts as x-ray marker to estimate a midline of a spine for cage or prosthetic disc replacement. The known and commonly used Caspar type distracter is then secured to the spine. There is however known use of distracter tubes that each slide over respective pins and which is secured to the pins via a screw applied at a threaded region . This however, does not have snug hex or square fit at a base of the pin but is round and transfers distraction forces along a length of a round pin. This does not provide optimal load transfer to the spinal vertebrae where distraction is required but rather applies the load at a moment arm distant from the required load application site. The known technique for distraction force application applies a bending and shear force to the pins which must be transferred down the pin to its point of engagement with the vertebrae. A mechanical advantage during distraction is provided the closer the load is applied to the vertebrae due to a reduced moment arm and elimination of a bending moment on the pins so it is desirable to provide an assembly which meets this objective. Accordingly, according to the description a shear force is applied at a base of pins to be distracted.

Throughout the specification a reference to a retractor arm may be taken to be synonymous with side arms, retractor blade and retraction member. A reference to blades will be taken to include a reference to retractors or retractor arms

To fully appreciate the various embodiments of the invention to be described below a summary of the bone fixation methodology and associated apparatuses and assemblies.

### 1. Bone fixation

### 1. Single point bone fixation

This may be accomplished by screws or short spikes or a spike / screw combination. Spikes are useful where a line of pull is oblique to a line of insertion and where anatomy is unfavorable for screws. Multiples screws or spikes may be used. A portion of the screw or spike may engage an d link to other components. This portion is referred to as the anchor.

Anchor recess shapes which may be used (see drawing/s) include the following non limiting components:
1. Mushroom (conical with enlarged point)
2. Cone
3. dimple
4. cup for blade
5. pedestal
6. Integral hinge and sidearm for connection to blade
7. Integral hinge allowing rotation in single plane
8. Integral small ball and socket with shaft for connection to blade.

### 2 Two point fixation with gripping devices

b. penetrating with 2 conical points (see figure 1 below)
c. non penetrating i.e. other clamping device with flat or curved face.

### 2. Means of rotation

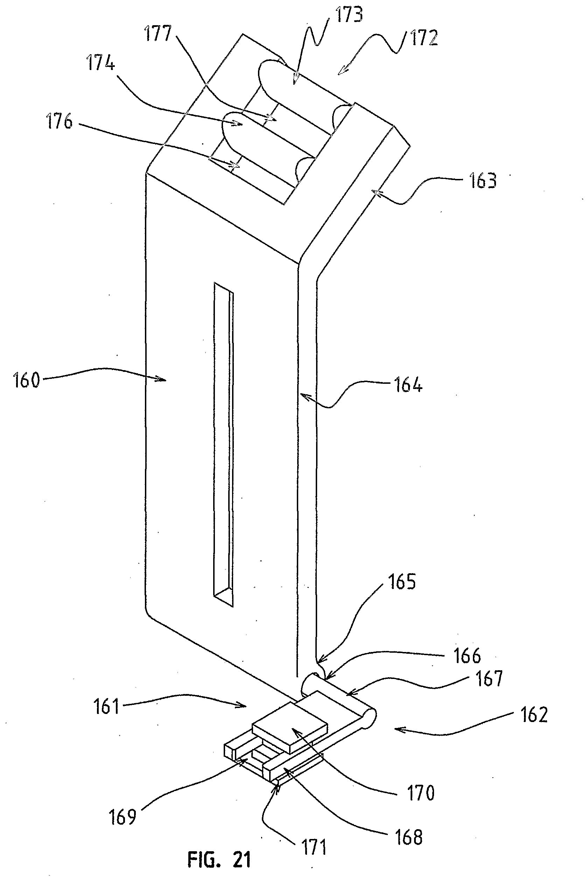

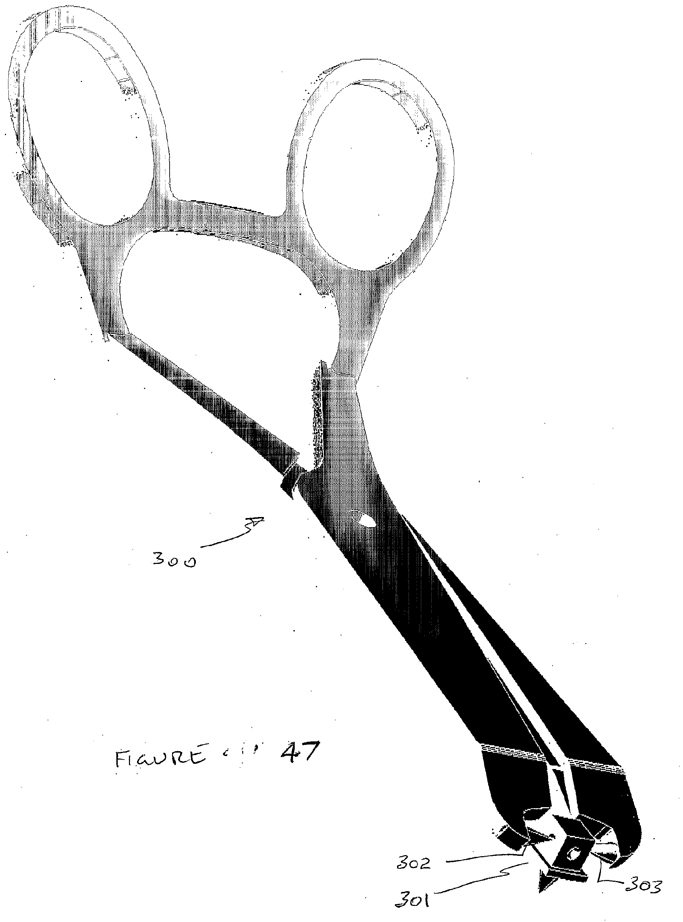

a. Anchor and blade and means of rotation are integral in one piece as shown below in figure 1.
b. Anchor incorporates means of rotation
   i. anchor with built in rotating shaft for blade attachment that allows rotation in 1 plane only.
   ii. Anchor with built in ball and socket plus shaft for blade fixation that allows rotation in multiple planes.
c. Anchor /blade interconnect directly e.g. dimple, mushroom, cone, cup, (see figures 47, 28, 37 and 44)
d. Link blade interface allows rotation either with integral or detachable hinge see figures 21, 31 , 33, 35 or as in. anterior cx system figure 20.

### Note on linking components

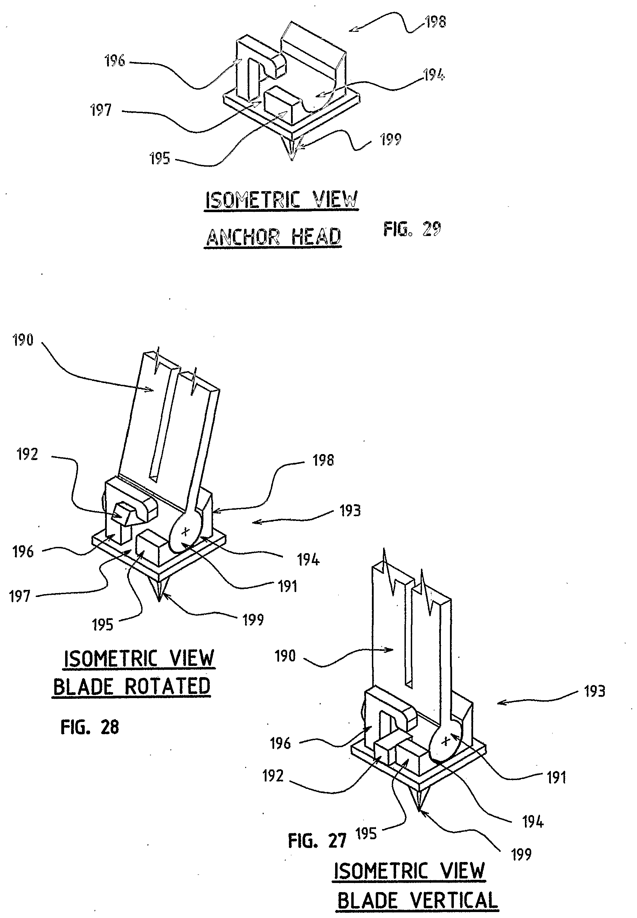

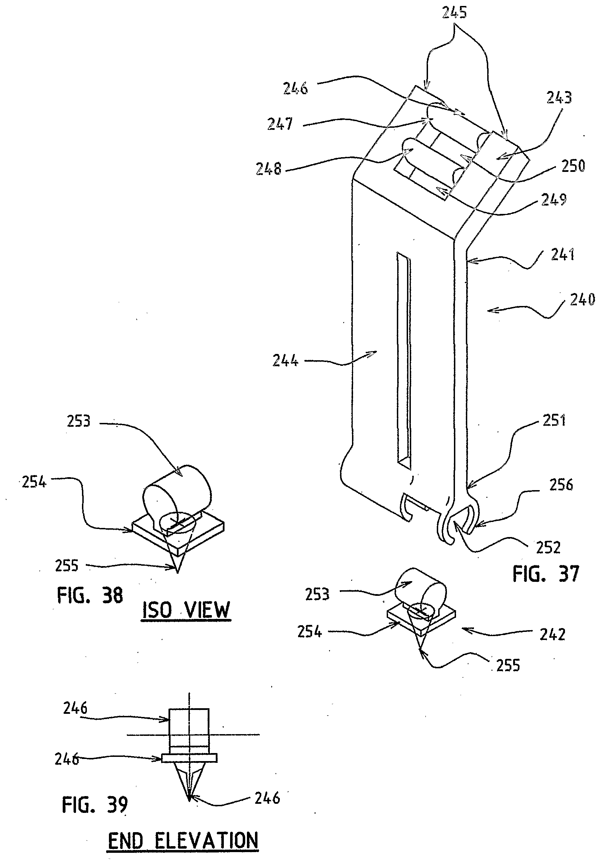

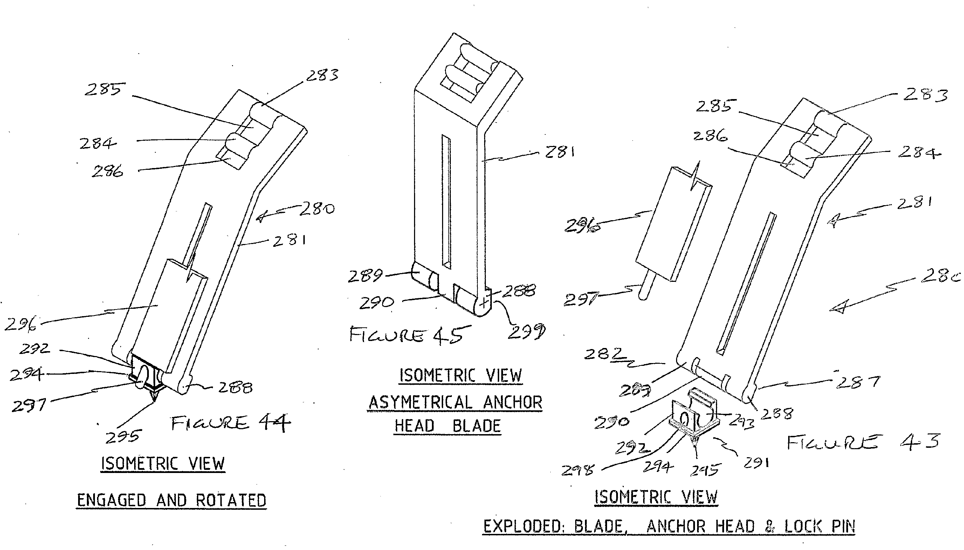

- It will be noted that the various combinations of component include methods for securing components together:
   a. That may permit desired free rotation but may limit movement in other planes likely to produce loosening or fall out.
      It will be obvious to those skilled in the field that the mechanisms shown limiting unwanted motion are additions and more simple versions of the following components with less stability are easily envisaged. flathead (Fig 43,44,45,46) which prevents lift out once rotated and restricts side to side movement
      ▪ modified cup with top loading and side loading blade with key lock feature **(****Fig 27, 28, 29****)** prevents lift out and side to side movement and limits rotation.
      □ modified cottage loaf **(****fig 37 and 38****)** and tunnel blade **(****Fig 37****)** prevents lift out and side to side movement
   b. That lock rotation at the desired point e.g.
      □ splines on sidearm and blade in anterior cx system **(fig nos 20, 40 - 42).**
      □ Flathead with recess for locking pin. Once retractor blade rotates to certain angle e.g. 30 degrees recess is exposed allowing pin to be inserted from above down front of the blade. This pin is easily pulled out allowing free rotation of the blade but by insertion prevents blade returning until pin removed. Locking the retractor blade in this fashion means external handles for the blades or weights to pull the blade outwards are avoided. This reduces number of instruments around operative field, frees or avoids totally the need for an assistant and facilitates x-rays. (**fig no 43 - 45)**
   c. There are known mechanism for "clicking" in that could be used to join blades from above onto secured anchors in situ. A retractor blade might thus click into position from above which is easier than sliding components together but once together resist pull out directly unless slid out sideways."

Referring to figure 1 there is shown a perspective view of an example of a retractor assembly 1. Retractor assembly 1 is a generally scissor action retractor comprising co operating scissor members 2 and 3 joined by pivot pin 4. Assembly 1 is operated by handle 5 including openings 6 and 7 which receive fingers of a user to effect the scissor action. Handle 5 further comprises a bridge 8 which allows relative movement between finger loops 9 and 10. To facilitate the relative movement, bridge 8 preferably includes an opposing ratchet allowing selective positioning of finger loops 9 and 10. Scissor member 3 terminates in a formation 11 comprising a recess 12 having therein a conical jaw 13. In the assemblies of figures 1, 20, and 40 a portion of a flat blade between the junction 4 of arms 2 and 3 (using figure 1 as example) and formation 14 constitute a flat surface which act as blade for purpose of tissue retraction as the retractor rotates about axis 18. The angulation between point 4 and formation 14 is for the purpose of improved visualization of the formation 14 during surgical fixation.

Scissor member 2 terminates in a formation 14 comprising a recess 15 having therein a conical jaw 16. Jaws 13 and 16 preferably lie along a common axis and are disposed in opposing relationship. Alternatively, jaws 13 and 16 may be disposed on parallel axes. Jaws 13 and 16 are conical each terminating in a sharp point adapted for engagement directly into bone or with an anchorage enabling rotation of assembly 1 about axis 17 in a rotational direction shown by arrow 18.

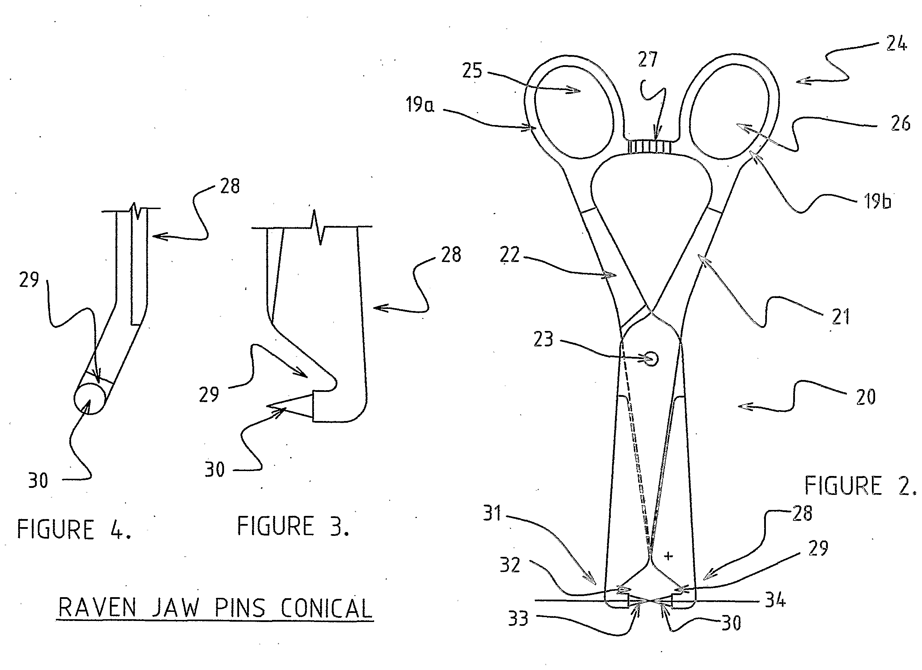

Referring to **figure 2** there is shown a perspective view of an example of a retractor assembly 20. Retractor assembly 20 is a generally scissor action retractor comprising co operating scissor members 21 and 22 joined by pivot pin 23. Assembly 20 is operated by handle 24 including openings 25 and 26 which receive fingers of a user to effect the scissor action. Handle 24 further comprises a bridge 27 which allows relative movement between finger loops 19a and 19b. This also contains a ratchet mechanism 27a to lock members 21 and 22 in position. Scissor member 22 terminates in a formation 28 comprising a recess 29 having therein a conical jaw 30. Scissor member 21 terminates in a formation 31 comprising a recess 32 having therein a conical jaw 33. Jaws 30 and 33 preferably lie along a common axis and are disposed in opposing relationship. Jaws 30 and 33 are conical each terminating in a sharp point adapted for engagement with bone directly or with an anchorage enabling rotation of assembly 20 about axis 34.

**Figure 3** shows an enlarged view of jaw pin 30 of the retractor assembly of figure 2. **Figure 4** shows a side elevation view of the jaw pin 30 of figure 3.

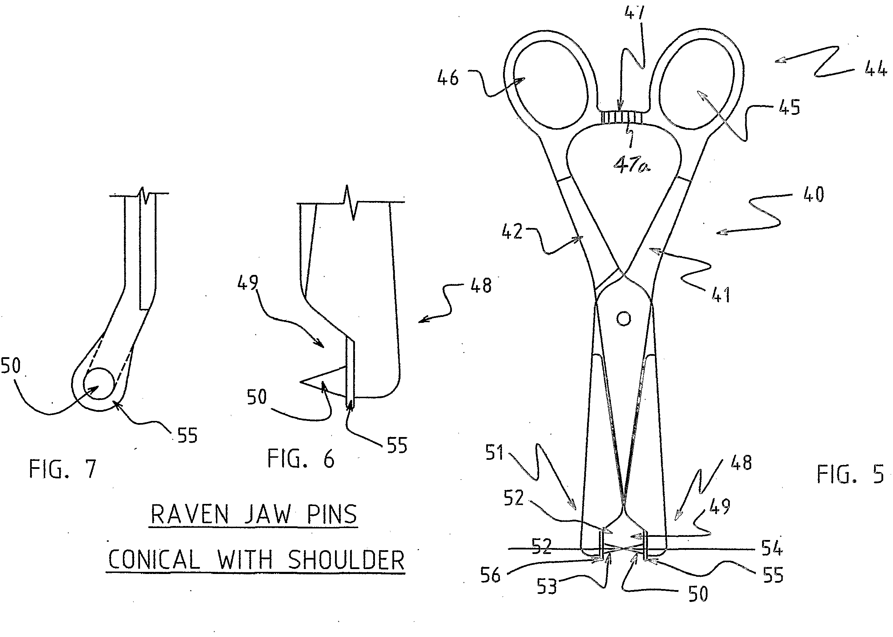

Referring to **figure 5** there is shown a perspective view of an example of a retractor assembly 40. Retractor assembly 40 is also a generally scissor action retractor comprising co operating scissor members 41 and 42 joined by pivot pin 43. Assembly 40 is operated by handle 44 including openings 45 and 46 which receive fingers of a user to effect the scissor action. Handle 44 further comprises a bridge 47 which allows relative movement between finger loops 45 and 46. This also contains a ratchet mechanism 47a. Scissor member 42 terminates in a formation 48 comprising a recess 49 having therein a conical jaw 50. Scissor member 41 terminates in a formation 51 comprising a recess 52 having therein a conical jaw 53. Jaws 50 and 53 are conical and preferably lie along a common axis and are disposed in opposing relationship. Jaws 50 and 53 each terminate in a sharp point adapted for engagement with an anchorage enabling rotation of assembly 40 about axis 54. Assembly is characterised in having flanges 55 and 56 at the base of respective conical jaws 50 and 53. Flanges 55 and 56 form respective shoulders on which an anchorage bears when assembly 40 engages an anchorage.

**Figure 6** shows an enlarged view of jaw pin 50 and flange shoulder 55 of the retractor assembly of figure 5. **Figure 7** shows a side elevation view of the enlarged jaw pin formation 48 of figure 6.

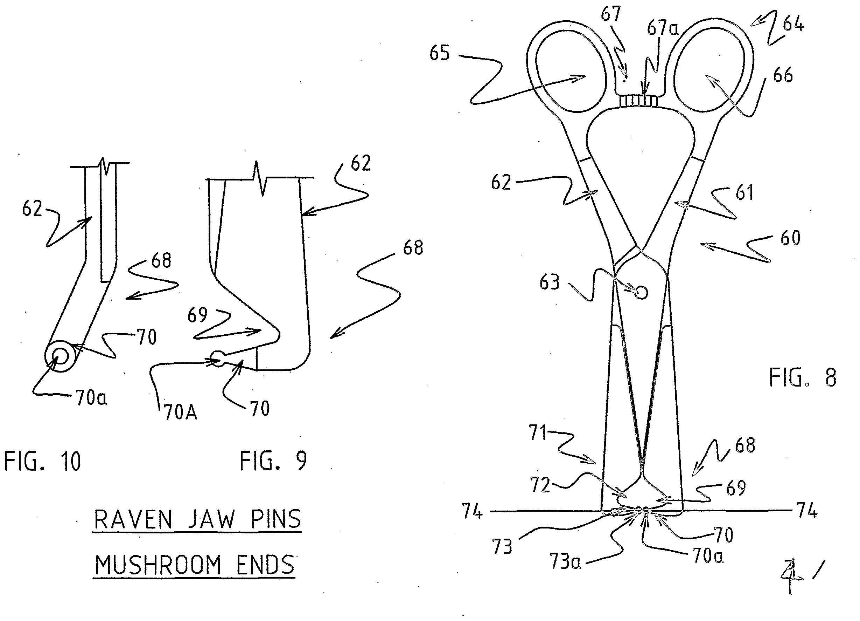

Referring to **figure 8** there is shown a perspective view of an example of a retractor assembly 60. Retractor assembly 60 is also a generally scissor action retractor comprising co operating scissor members 61 and 62 joined by pivot pin 63. Assembly 60 is operated by handle 64 including openings 65 and 66 which receive fingers of a user to effect the scissor action. Handle 64 further comprises a bridge 67 which allows relative movement between finger loops 65 and 66 preferably by ratchet adjustment 67a. Scissor member 62 terminates in a formation 68 comprising a recess 69 having therein a jaw 70. Scissor member 61 terminates in a formation 71 comprising a recess 72 having therein a jaw 73. Jaws 70 and 73 have enlarged mushroom like points 70a and 73a respectively and preferably lie along a common axis and are disposed in opposing relationship. Enlarged mushroom like points 70a and 73a are adapted for engagement with an anchorage enabling rotation of assembly 60 about axis 74. Assembly 60 is essentially characterised by the blunt mushroom formations 70a and 73a.

**Figure 9** shows an enlarged view of jaw 70 of the retractor assembly 60 of figure 8. **Figure 10** shows a side elevation view of the enlarged jaw 70 of formation 68 of figure 9.

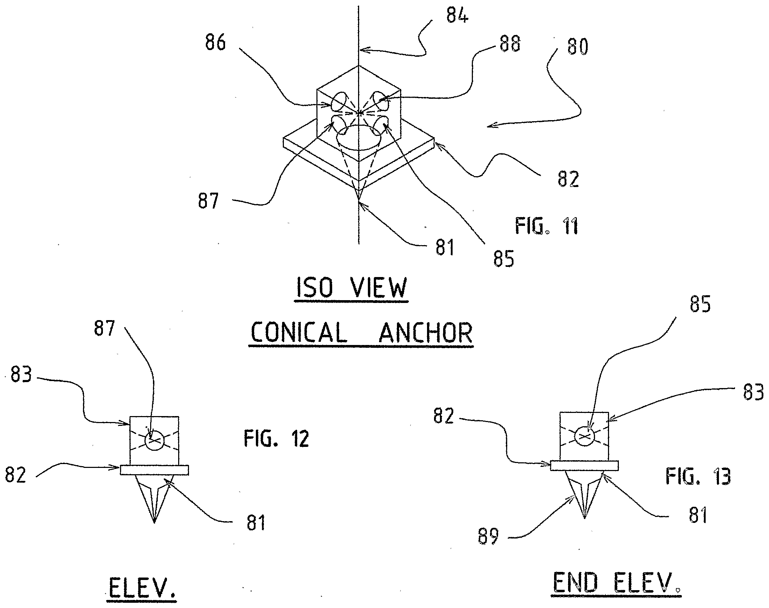

**Figure 11** shows a perspective view of an example of a retractor assembly conical anchor. Anchor 80 comprises an anchor pin 81 depending from base 82. Base 82 supports an anchor head 83. Axis 84 is co linear with pin 81 and head 83. Head 83 includes conical recesses 85 and 86 and normally disposed thereto recesses 87 and 88. Anchor 80 is ideally suited for use with the retractor assemblies 1, 20 and 40 of figures 1 , 2 and 5 respectively. According to one example, pin 81 includes formations 89 which facilitate penetration into a bony anchorage. **Figure 12** shows an elevation view of the anchor 80 of figure 11 with corresponding numbering. **Figure 13** shows an end elevation of the anchor 80 of figure 11.

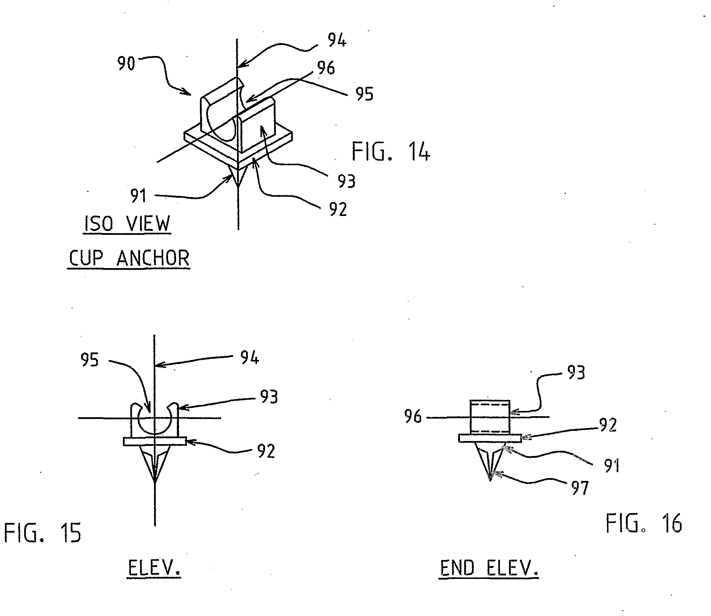

**Figure 14** shows a perspective view of an example of a retractor assembly cup anchor. Anchor 90 comprises an anchor pin 91 depending from base 92. Base 92 supports an anchor head 93. Axis 94 is co linear with pin 91 and head 93. Although all examples drawn show formation for an engaging anchor with bone being co-linear with an anchor head, it is envisaged that anchorage pins or screws may be at an angle to an anchor head and could be used for fixation of the anchor to bone where anatomy of bone is more vertically orientated eg around an acetabular cup in hip joint surgery. Screwdrivers for inserting screws in a direction not co linear with a screwdriver are known and could be employed. Simple impaction of a pin portion of an anchor is also envisaged. Formation of an anchor for engaging bone could also rotate in simple hinge or ball and socket configuration as part of an anchor itself allowing vertical alignment of an anchor head even if formation for engaging bone is not vertical. Head 93 includes a cup recess 95 having its longitudinal axis 96 transverse to axis 94. According to one example, pin 91 includes formations 97 which facilitate penetration into a bony anchorage. Anchor 90 is ideally suited for use with the retractor arm as shown in figures 43 - 45. In practice the anchor 90 would be inserted into bone at the desired position in bone using awl and screw driver or if pin direct hammering. A retractor blade such as that shown in figure 28 is then slid in laterally to engage anchor head 93 and this rotates in recess 95 with recess engaging a corresponding round portion of the blade to enable smooth rotation and also to prevent lift out. Variable rotation with stability thus accomplished.

**Figure 15** shows an elevation view of the anchor 90 of figure 14 with corresponding numbering. **Figure 16** shows an end elevation of the anchor 90 of figure 14.

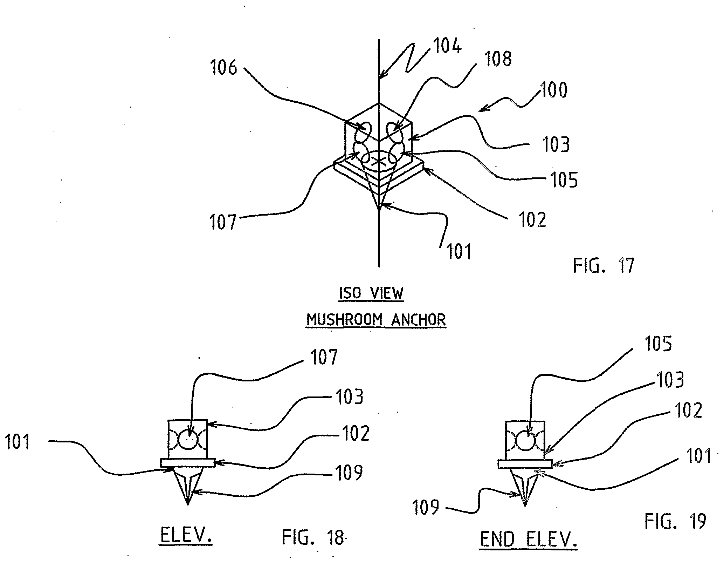

**Figure 17** shows a perspective view an example of a retractor assembly mushroom **anchor** 100. Anchor 100 comprises an anchor pin 101 depending from base 102. Base 102 supports an anchor head 103. Axis 104 is co linear with pin 101 and head 103. Head 103 includes recesses 105 and 106 and normally disposed thereto recesses 107 and 108. Anchor 100 is ideally suited for use with the retractor assembly 60 of figures 8, 9 and 10 respectively. According to one example, pin 101 includes formations 109 which facilitate penetration into a bony anchorage Figure 18 shows an elevation view of the anchor 100 of figure 17 with corresponding numbering. **Figure 19** shows an end elevation of the anchor 100 of figure 11.

**Figure 20** shows a perspective view of a retractor assembly capable of concurrent retraction and distraction by co operating anchor pins according to a preferred embodiment of the invention. Referring to figure 20 there is provided a retraction and distraction assembly 110 comprising retractor arms 111 and 112 terminating respectively in joining members 113 and 114 which engage anchor pin assemblies 115 and 116. Various shapes of retractor joining member / side arms are possible as required. Retractor arm 111 comprises a first free end portion 117 including a recess 118 and spanning therebetween bridge members 119 and 120. Bridge members 119 and 120 define spaces 121 and 122 which may attach to an operating tool not shown. The operating tool facilitates rotational movement of retractor arm 111. Retractor arm 112 comprises a first free end portion 123 including a recess 124 and spanning therebetween bridge members 125 and 126. Bridge members 125 and 126 define spaces 127 and 128 which may receive an operating tool not shown. As with arm 111, the operating tool facilitates rotational movement of retractor arm 112 such that arms 111 and 112 co operate in maintaining retraction of opposing surgical wound margins. Arms 111 and 112 are in use disposed against opposing soft tissue wound margins during surgery to facilitate and maintain adjustable rotational retraction. Arm 111 further comprises blade region 129 which terminates in a formation 130 which defines a recess 131 which receives and retains therein joining arm 132 of joining member 113. This allows arm 111 to selectively rotate about joining arm 132 . Joining member 113 also comprises a bifurcated portion 133 having two arms which define recess 134 which receives and retains anchorage assembly 115. Anchorage assembly 115 comprises an anchorage pin 135 having a leading end 136 terminating in a sharp point 137. Leading end 136 may have thread like screw or pin like to facilitate penetration in bone and a trailing end 138 providing a driving member 138a to facilitate bone penetration of point 137. Pin 135 is housed in guide sleeve 139 which compresses bifurcated portion 133. Joining member 113 locates between base 138a and end 139a of sleeve 139. Guide sleeve 139 has connected thereto an ancillary sleeve 140 which receives in recess 141 a tool (not shown) to facilitate distraction i.e. separation of anchorage assemblies 116 and 115 along an axis usually but not exclusively parallel to the axes of rotation of arms 111 and 112 . Arm 112 further comprises blade region 112a which terminates in a formation 142 which defines a recess 143 which receives and retains therein joining arm 144 of joining member 114. This allows arm 112 to selectively rotate about joining arm 144. Joining member 114 also comprises a bifurcated portion 145 having two arms which define recess 146 which receives and retains anchorage assembly 116. Anchorage assembly 116 comprises an anchorage pin 147 having a leading end 148 terminating in a sharp point 149 to facilitate penetration in bone and a trailing end 150 providing a driving member 151 to facilitate compression of 152 against 145 securing joining member 114. Pin 147 is housed in guide sleeve 152 which engages bifurcated portion 145. Guide sleeve 152 has connected thereto an ancillary sleeve 153 which receives in recess 154 a tool (not shown) to facilitate distraction of anchorage assemblies 116 and 115 along an axis parallel to the axes of rotation of arms 111 and 112 . The sleeves 152 and 139 engage onto square portions of respective pins. The bifurcated elements 133 and 145 also engage snugly around square portions of pins 135 and 147. This connection prevents independent rotation of elements. Combined rotation of all elements is prevented by joining two sleeves and pins together with an external distraction assembly. This distraction assembly (not shown) may join with the assembly as shown via recess 154 and 158 or may be integral as in existing Caspar systems.

With the above described assembly 110 a surgeon may selectively effect both distraction (of bone) and retraction (of soft tissues). The assembly allows distraction along an axis parallel to the axes of rotation of arms 111 and 112 and selective opposing rotational adjustments of arms 111 and 112 and if required removal of one or other of arms 111 and 112.

**Figure 21** shows a perspective view of a part retractor assembly showing retraction arm 160 anchored to an anchor pin 161 by a joining member 162 according to an alternative embodiment of the invention. Arm 160 comprises free end portion 163 and blade region 164 which terminates in a formation 165 which defines a recess 166 which receives and retains therein joining arm 167 of joining member 162. This allows retraction arm 160 to selectively rotate about joining arm 167. Joining member 162 also comprises a bifurcated portion 168 having two arms which define recess 169 which releasably receives and retains head 170 of anchorage pin 161 terminating in a sharp point 171 to facilitate penetration in bone. Free end portion 163 includes recess 172 which retains bridge members 173 and 174. Bridge members define openings 176 and 177 which receive an operating tool to facilitate both selective rotation of arm 160 and detachment from either joining member 162 or anchor 161.

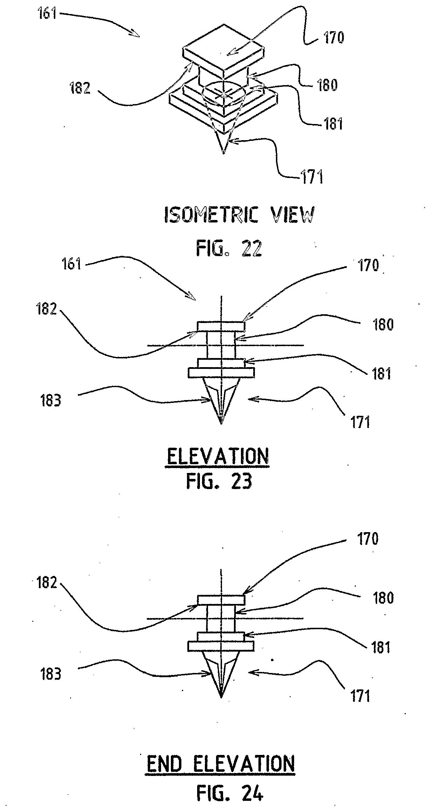

**Figure 22** shows an enlarged view of the retractor arm anchor pin 161 of figure 21 with corresponding numbering. Head 170 includes recess 180 which receives bifurcated portion 168 of joining member 162. Bifurcated portion 168 bears on shoulder 181 and locks against underside surface 182 of head 170. Shoulder 181 is optional and could be removed allowing bifurcated portion 168 of 162 to be positioned securely between 182 and base plate 170a.

**Figure 23** shows an elevation view of the arm anchor pin 161 of figure 22 with corresponding numbering. Point 171 may further comprises formations 183 which facilitate bone penetration. **Figure 24** shows an elevation view of the arm anchor pin 161 of figure 22. This arrangement of blade secured to anchor pin 161 with mechanism permitting rotation has widespread application in but not limited to orthopaedic and spinal surgery and whenever bone fixation possible. Variations of such devices will be obvious to those skilled in the art.

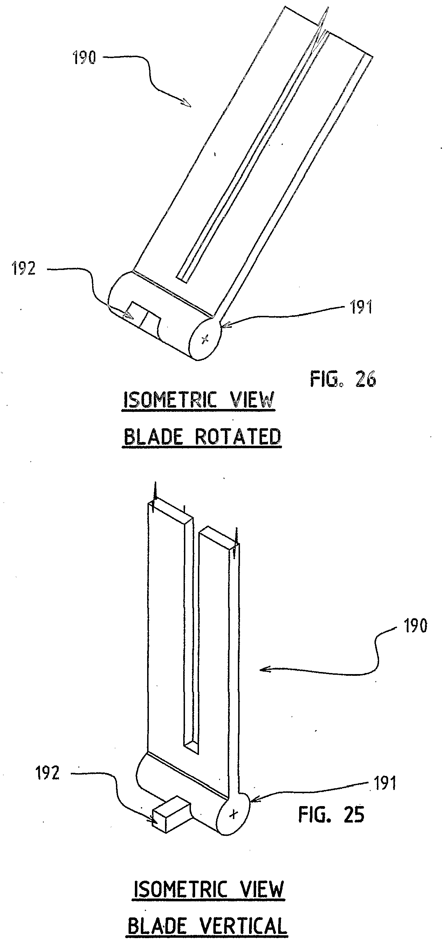

**Figure 25** shows a retractor arm 190 according to an alternative embodiment of the invention disposed in a substantially vertical orientation. Arm 190 terminates in a formation 191 which includes a locking tang 192. **Figure 26** shows the retractor arm 190 of figure 25 rotated clockwise from the vertical.

**Figure 27** shows a retractor arm and anchorage pin assembly 193 according to an alternative embodiment of the invention with the retractor arm 190 of figures 25 and 26 in a substantially vertical orientation. Anchorage pin assembly 193 comprises a cup recess 194 which receives formation 191 allowing relative rotation between cup recess 194 and arm 190. **Figure 28** shows the retractor arm 190 of figure 27 rotated clockwise from the vertical. Pin assembly 193 further comprises first abutment 195 and second abutment 196 defining a space therebetween 197. In figure 27 locking tang 192 engages abutment 195 and locates in space 197. This occurs when arm 190 is vertical and in longitudinal alignment with pin 199. Locking tang 192 engages abutment 196 when arm 190 is rotated 30 degrees. Thus abutment 196 may be configured according to the maximum extent of rotation required for arm 190. Arm 190 may be released from anchorage assembly 193 by anti clockwise rotation from the disposition of figure 28 to that shown in figure 27. Arm 190 may then be lifted up and slid laterally to free locking tang 192 from abutments 195 and 196. **Figure 29** shows the anchorage pin assembly 193 of figures 27 and 28 with retractor arm 190 released from recess 194 of the anchor head 198. This arrangement permits secure linkage of components while allowing rotation to desired degree. Top and side loading of blade to anchor is achieved.

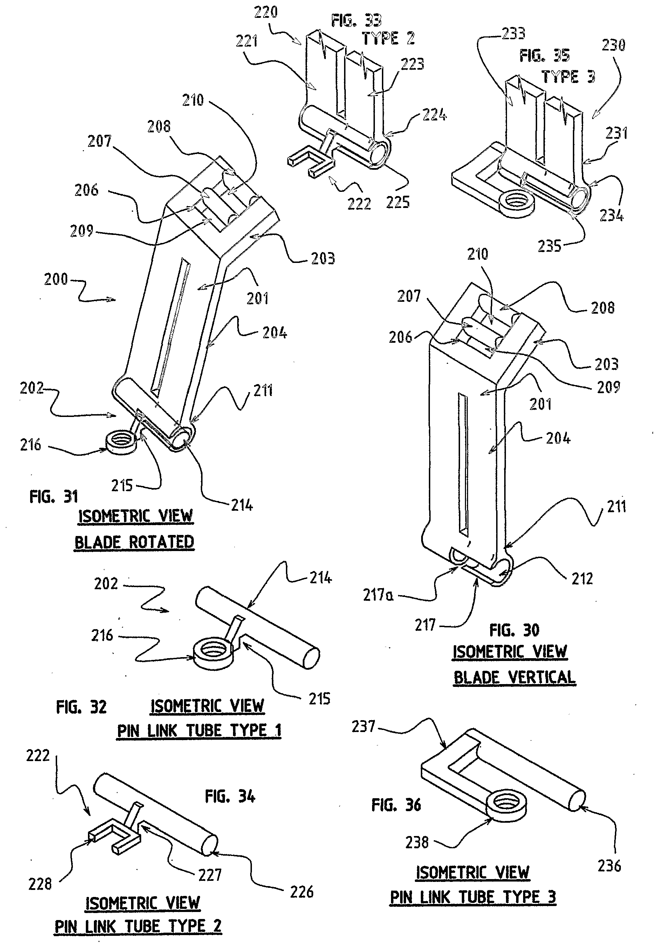

**Figure 30** shows the retractor arm 201 of figure 31 separated from a joining member **202.**

**Figure 31** shows a retractor arm and joining member assembly 200 according to an alternative embodiment of the invention. Assembly 200 comprises a retractor arm 201 and joining member 202 with the retractor arm 201 in a substantially vertical orientation. Arm 201 includes free end portion 203 and blade 204. End 203 includes bifurcation 205 defining recess 206. Recess 206 includes bridging members 207 and 208 defining recesses 209 and 210 which receive an operating tool (not shown) which in use facilitates arm rotation for retraction of soft tissues. Blade portion 204 terminates in an enlarged formation 211 defining a recess 212 having a partially cut away wall exposing the interior of recess 212. This cut away allows insertion and removal of members 202 and 222 plus rotation once centered . Recess 212 receives and retains joining member 202.

**Figure 32** shows an enlarged view of the joining member of figure 31. Joining member 202 is insertable in recess 212 via pivot arm 214. Pivot arm 214 has preferably intermediate its ends a bridge 215 which terminates in a loop 216 which engages an anchor pin (not shown). Arm 201 may then be rotated through about 30 degrees but it will be appreciated that the wall 217 (see figure 30) contains notch 217a that allows rotation of bridge 215 to occur limiting rotation as desired. of recess 212 can be configured to achieve an alternative degree (more or less) of rotation.

**Figure 33** shows a retractor arm and joining member assembly 220 according to an alternative embodiment of the invention. Assembly 220 comprises a retractor arm 221 and joining member 222 with the retractor arm 221 abbreviated but in a substantially vertical orientation. Blade portion 223 terminates in an enlarged formation 224 defining a recess 225 having a partially cut away wall exposing the interior of recess 225. **Figure 34** shows an enlarged view of the joining member of figure 33. Recess 225 receives and retains pivot arm 226 about which retractor arm 221 is free to rotate. Pivot arm 226 has preferably intermediate its ends a bridge 227 which terminates in an open saddle 228 which engages an anchor pin (not shown). Arm 221 may then be rotated as required about pivot arm 226.

**Figure 35** shows a retractor arm and joining member assembly 230 according to an alternative embodiment of the invention. Assembly 230 comprises a retractor arm 231 and joining member 232 with the retractor arm 231 abbreviated but in a substantially vertical orientation. Blade portion 233 terminates in an enlarged formation 234 defining a recess 235 having a partially cut away wall exposing the interior of recess 235. **Figure 36** shows an enlarged view of the joining member of figure 35. Recess 235 receives and retains pivot arm 236 about which retractor arm 231 is free to rotate. Pivot arm 236 has preferably at one end a bridge 237 which terminates in an open saddle 238 which engages an anchor pin (not shown). Retractor arm 231 may then be rotated as required about pivot arm 236.

**Figure 37** shows an exploded view of a retractor arm and anchor member assembly 240 according to an alternative embodiment of the invention. Assembly 240 comprises a retractor arm 241 and anchor member 242. Arm 241 includes free end portion 243 and blade 244. End portion 243 includes bifurcation 245 defining recess 246. Recess 246 includes bridging members 247 and 248 defining recesses 249 and 250 which receive an operating tool (not shown) which in use facilitates arm rotation for retraction of soft tissues. Blade portion 244 terminates in an enlarged formation 251 defining a recess 252 having a partially cut away wall exposing the interior of recess 252. Recess 252 receives and retains anchor member 242. **Figure 38** shows an enlarged view of the anchor member 242 of figure 37. Anchor member 242 comprises anchor head 253 mounted on base 254. Anchor pin 255 depends from base 254 and in use penetrates bone. Anchor member 242 is insertable in recess 252 of arm 241 via anchor head 253. Arm 241 rotates about anchor head 253 which is cylindrical to facilitate rotation of arm 241. Blade portion 244 includes a bifurcated end retainer 256 which provides an abutment against which head 253 bears. **Figure 39** shows an end elevation of the anchor pin of figure 38.

These variations permit attachment of retractor blades close to where required allowing secure fixation and rotation. Variations depending on anatomy are required but the principle remains the same : namely, that a secure axis of rotation is achieved from within a surgical wound. Components can be separated and assembled prior to insertion by the scrub nurse or inserted separately and joined in situ. Disassembly facilitates cleaning, sterilisation and replacement of components. Various materials in medical usage for fabrication are envisaged.

According to an alternative embodiment of the invention, the retraction assembly uses a single pin as a point of fixation for retractor blades but without a second pin. This would utilize a single tube not connected to distracter mechanism for surgeons not wishing or able to use distraction.

A retraction arm also described as a side arm co operates with a blade portion to perform retraction of soft tissue wound margins.

Joining members which engage retractor blades (also referred to in the art as side arms) may be flat in one plane or with a bend so that part lies in one plane and another part in another plane allowing rods to sit a few mms below the plane assumed by the jaws of the bifurcated elements to fit a natural contour of a spine.

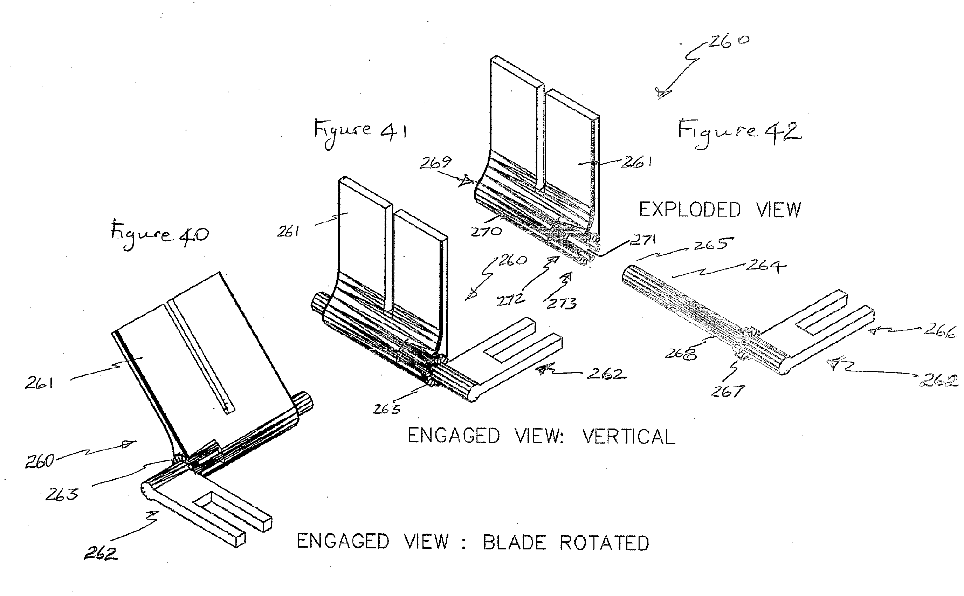

Flat members of the type shown in Figure 40 are reversible and can be used from top or bottom. Bent joining members / side arms are still reversible but top left could only be used as bottom right and vice versa. Surgeons may choose whether to use two side arms at top i.e. one to left and one to right or one sidearm at top to one side and one from bottom to other. For surgery involving two disc levels, two joining members / side arms may be used from the top and two from the bottom providing longer area of exposure.

Side arms allow rotation while maintaining a strong join to avoid disconnection and maintaining point of fixation of retractor blades down on a spine where required. Male/female connection with rod on joining member / side arm and tube on retraction arms at the base of retraction blades is preferred. Splines (see figures 40 - 42) on the rods 132 and 144 and mating formations on 130 and 142 of the retraction arms 111 and 112 prevent unwanted rotation during insertion while allowing the surgeon to adjust the angle between the retractor sidearm and retractor blade for ease of insertion. Once inserted, the blade may be withdrawn slightly axially along the rod disengaging the splines (see below) . This allows the blades 111 and 112 to be rotated to a desired position and then reinserted locking in position. This may avoid having to apply any additional surface retractor mechanism.

A tendency of the retractor blade and retractor joining member side arm to rotate pins is resisted by dual tubes which once fitted, will prevent independent movement of screws, side arms and blades.

According to one embodiment splines are used to make the engagement between the retractor arms and retractor blades but this is a non limiting engagement arrangement and it will be appreciated by persons skilled in the art that other forms of engagement which preferably accommodate axial release and locking and also selective rotation may be employed. For example, a ratchet mechanism may be used. The retraction blades are drawn according to one embodiment, with splines but this is to be taken as a non limiting arrangement. Not all combinations of flat or offset, open or closed, with or without splines adjustable or non adjustable are shown. Numerous shapes are envisaged to accommodate different areas of the spine (and other parts of the body e.g. in non spinal orthopaedics.)
Referring to **figure 40** there is shown a perspective view of an assembly 260 including abbreviated retractor arm 261 and joining member 262 including locating spline assembly 263 according to one embodiment of the invention. Figure 40 shows arm 261 at an oblique angle relative to a horizontal plane through joining member 262. **Figure 41** shows the arrangement of figure 40 with arm 261 rotated to an alternative position disposed normally to the plane of joining member 262. **Figure 42** shows an exploded view of the assembly 260 of figure 40. Joining member 262 includes a joining shaft 264 terminating in a free end 265 and having a knurled or bevelled surface. Member 262 terminates at its opposite end in a bifurcated member 266 which engages an anchor (not shown ) similar to the arrangements previously described. Shaft 264 includes thereon a radial array of splines 267 extending from its circumferential surface 268. Arm 261 terminates in an enlarged region 269 having a wall 270 which defines a recess 271 adapted to axially receive shaft 264. Wall 270 has at one end 272 a radial array of elements 273 arranged to align with intermediate spaces between individual splines in spline array 267. This arrangement allows arm 261 to be selectively released from spline array 267 rotated to alternative positions such as shown in figures 40 and 41. This allows a surgeon to selectively position the angle of repose of arm 261 held securely in position by splines 267. To reposition the angle of arm 261 this is axially released from splines 267, rotated and then reset. Splines provide a strong resistance to rotational loads applied against the arm 261 during use as a retractor.

In an alternative embodiment it will be appreciated that position of spline on shaft 268 may be varied to opposite end 265 with a corresponding change of mating profile to other end of recess 271 and in a further embodiment blade could contain splines and the shaft the recesses.' Opposite gender spline mating may also be employed.

The number of splines and teeth angles may be varied according to requirements. In an example of a method of use of the assembly, not forming part of the invention, a surgeon may assemble the combined distracter and retractor assembly of figure 20 according to the following regime. Typically, the assembly may be employed in an anterior approach to the cervical spine. The first step which is based on a known technique involves insertion of pins in vertebrae to be distracted. Preferably there will be two pins 135 and 147 spaced apart which are distracted to allow access to a disc space in the case for instance where a disc is to be replaced. The distracter pins such as those described as in figure 20 are inserted into adjacent vertebrae. The surgeon takes a first retractor arm 111 or 112 whose configuration is selected to accommodate patient anatomy and allows it to directly or indirectly engage a first of said pins causing an associated rod and blade to lie generally parallel to a longitudinal axis of a spine. Arms 111 and 112 are mounted respectively on arms 132 and 144 of joining members 113 and 114. Sleeves 139 and 152 are positioned over respective pins 135 and 147. A retractor blade 111 or 112 such as that described with reference to figure 20 is attached if not previously done by sliding respective sleeves (130 and 142) on the blade along the pivot arm of a joining member 132, 144 until mating splines (or other mechanically equivalent engagement) engage. The orientation of the retractor blade is set according to a selected circumferential (rotational) engagement of the respective mating opposed splines. The assembly now formed and shown in figure 20 provides an ability to both distract vertebrae and retract soft tissue as required, the distraction occurring when a distraction force is applied through the sleeves or tubes 139 and 152 which axially engage the pins 136 and 147.

The degree of retraction is set by engaging the splines at a predetermined position so as to set the retractor arms 111 and 112 at an angle of repose which keeps soft tissue margins apart as required by the surgeon. If the aforesaid description relates to a right side distracter arm and retractor blade assembly there will be a corresponding left hand side arrangement which is preferably symmetrical about a transverse line through a disc space. If the aforesaid description relates to a left side distracter arm and retractor blade there will be a corresponding right hand side arrangement which is again symmetrical about a transverse line through the disc space. The distraction forces are applied at the base of the pins 136 and 147 to optimise mechanical advantage and to eliminate pin bending loads. This assembly described above allows variable distraction poses and movement without removal of retractors - side retractor arms and blades.

According to one aspect of the disclosure once the retractor blades are inserted they are fixed at or adjacent to a location where a tip of an end of the Retractor blade is required. This is usually deep in the wound and generally involves firm fixation to an adjacent bony surface via a screw, clamp or other gripping device that connects directly to the blade or via an intermediary linkage.

In most situations this connection with the fixation device will allow rotation of the blade about the point of fixation, either because the point of fixation itself can rotate, e.g. semicircular spikes forming a pincer or via an intermediary linkage which allows the rotation while remaining in fixation as in the cervical assembly described in detail. This fixation with rotation allows retractor blades to be left insitu throughout a procedure but allowing the surgeon to release the pressure and the retraction forces while working on another area thus reducing the tissue trauma but without having to remove the retractor blade or reposition the retractor blade. Releasing self retaining retractors leads to repeated tissue trauma every time these are reinserted and/or reopened. This system would therefore reduce tissue trauma and save time. It would also mean that the retractor once appropriately inserted can be secured insitu and not become loose or dislodged and require repositioning. The fixation device described in the cervical assembly utilises a pin with a screw thread into the bone.

An alternative example of a retractor assembly such as that described in figure 1 uses a variety of fixation devices including pincer type spikes on a curved, angled or straight scissor type handle which grip irregular or curved bony surfaces and then rotate about the pincer grip the retractor blade being attached to the arms or hinges of this device. This would be used in one example in the posterior lumbar spinal surgery where flat surfaces are less available or bone available for fixation is insufficient depth or strength to support a screw fixation. In one example the assembly in figure 1 is directly fixed to top and bottom sides at a base of the transverse processes in the lumbar vertebral body. The rotating blade acts as a retractor to aid placement of pedicle screws. Use of a bone anchor with modified device as in figure 47 would aid exposure of the sacrum for sacral pedicle screws. Use of bone anchors with a blade directly as in figures 21, 27, 31 and 37 have multiple applications. Cup anchors (fig 14) allow a matched shaped lower portion of a blade to engage/disengage from the side ie slide across and then freely rotate. More complex versions of this allowing loading from above, restriction of unwanted motion and locking include those shown in fig 28, 37 and 44 which by limiting motion improve stability of connection between anchor and respective blade. This combination of components has multiple applications for the purpose of retraction of tissues. Such anchors could also act as components in distraction devices alone or be combined with function as base for rotating retractors as illustrated in fig 20.

Figure 43 shows an exploded view of a retraction arm and anchorage assembly 280 according to an alternative embodiment of the invention. Assembly 280 comprises retractor arm 281 which apart from its distal end 282 has similar geometry and operational characteristics to the retractor arms previous described herein. Arm 281 comprises a proximal portion having bridge members 283 and 284 which define recess 285 and 286 adapted to receive an adjustment tool not shown. Arm 281 terminates at distal end 282 in a formation 287 comprising substantially cylindrical ends 288 and 289 and a flat region 290 intermediate therebetween. Assembly 280 also comprises an anchor 291 having an anchor head 292 including a recess 293, supported on base 294 and a depending anchor pin 295. Anchor pin 295 may be hammered into bone or alternatively screwed in. The assembly further comprises a locking plate 296 terminating in a locking pin 297.

**Figure 44** shows an assembled view of the retraction arm and anchorage assembly 280 of figure 43. In this arrangement assembly 280 is shown with arm 281 engaged with anchor 291 and locking plate 296 engaging arm 281 via formation 287 at distal end 282. Cylindrical ends 288 and 289 protrude from recess 293 and flat region 290 intermediate therebetween engages recess 293. Locking pin 297 engages oblique recess 298 (see figure 43) to lock arm 281 against rotation. To release arm 281 from anchor 291 locking pin 297 is simply extracted from recess 298. One advantage of this arrangement is that arm 281 can be inserted and removed within a narrow corridor . Another advantage is that there is no need to release arm 281 laterally (axially) from a shaft which requires a larger working space. **Figure 45** shows arm 281 in isolation . It can be seen from this figure that substantially cylindrical end 288 has a flat region 299. The flathead anchor is secured to bone. The blade is positioned with section 290 directly above recess 293. The opening of the recess in the anchor head 293 is such that it engages the leading edges of flattened sections of the blade 290 allowing the blade to slide into position only when the blade is positioned at a set angle. Once fully engaged the blade can rotate. The amount of rotation is restricted by limiting abutment surfaces of blade and anchor head. The rotation brings the larger diameter circular portions of the section 290 under the recess opening preventing it lifting out. It can only be removed by returning to position of entry from where it can be lifted out. The more circular side portions 289 of lower aspect of blade prevent it sliding sideways out of position once engaged. These are optional and the entire base of the blade may have a flat head if desirable to have more side to side freedom. The angle of entry would be set to ease entry while preventing accidental fall out when under retraction or when retraction relaxed. The recess in the anchor head is optional and for a matched locking pin. Once the blade is rotated to a set position this reveals the entire recess and allows insertion of the pin that completely locks the blade in position relative to anchor. Variations in configurations of pin, recess and blade would allow locking at different degrees of rotation as desired. This significant addition avoids the need for external weights or continued external retraction pressure to maintain the position of the blade and is desirable by reducing instruments or assistants required during a surgical procedure. Rails or grooves in the blade to facilitate insertion of pin and attachment to blade are simple additions and obvious to persons skilled in the art.'

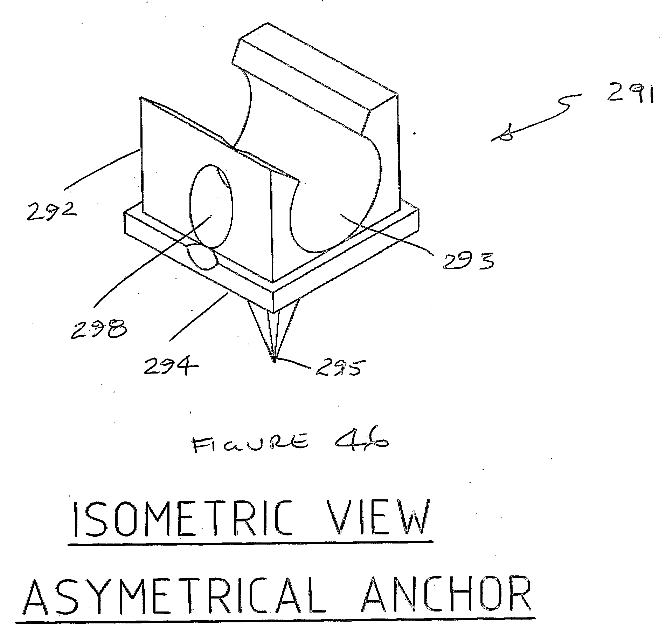

**Figure 46** shows a perspective view of anchor 291 showing oblique recess 298.

Figure 47 shows a perspective view of a retractor assembly 300 of the type described in figure 1 engaging an anchor 301. Opposing conical pins 302 and 303 engage corresponding recesses in anchor 301.

It will be appreciated by those skilled in the art that the utilisation of this principle could be used in numerous other applications adapting to the different anatomy and retraction requirements throughout the spine, musculoskeletal system or wherever bony fixation can be utilised, e.g. the head.

It will be further recognised by persons skilled in the art that numerous variations and modifications may be made to the invention without departing from the overall scope of the invention as defined in the appended claims. Such modifications would allow adaptation of key concepts (which is that retractor blades are fixed at or close to critical point of retraction and may rotate) to provide additional retraction devices for use in anterior or posterior spinal surgery throughout the length of the spine or in orthopaedics or other surgical disciplines where bony fixation is available. The invention described herein relates to the distraction and retraction apparatus and not to the surgical method of the operations in which those apparatus are used.

## Claims

1. An assembly capable of retraction of soft tissue and distraction of bone during spinal surgery, wherein the assembly comprises:
an anchorage including;
a first joining member (113) which co operates with a first anchor pin (135) having a distal end (137) which engages vertebral bone and which anchors the first joining member (113); and
a second joining member (114) which co operates with a second anchor pin (147) having distal end (148) which engages vertebral bone and which anchors the second joining member (114);
wherein relative movement between the first and second joining members (113, 114) is allowed during bone distraction distraction through said anchor pins (135, 147);
and wherein the first joining member (113) includes a retaining arm (132) which receives and retains a first retraction member (111) having a first free end and a second end, the second end including a formation (130) which allows the first retraction member (111) to be retained on the retaining arm (132) and move along the retaining arm (132) during distraction of bone through the anchor pins (135, 147).

2. An assembly according to claim 1 wherein the second joining member (114) comprises a second retaining arm (144) which receives and retains a second retraction member (112) having a first free end and a second end, the second end including a formation (142) which allows the second retraction member (112) to be retained on the arm; wherein said first and second retraction members (111, 112) co operate to retract soft tissue while said first and second anchor pins (135, 147) co operate to effect said distraction of bone.

3. An assembly according to claim 2 wherein, each retraction member (111, 112) is rotatable about respective retaining arms (132, 144).

4. An assembly according to claim 3 wherein, the retraction members (111, 112) are detachably retained on the retaining arms (132, 144).

5. An assembly according to claim 4 wherein, each retaining arm (132, 144) provides an axis of rotation for one said retraction members (111, 112).

6. An assembly according to claim 5 wherein, the first and second joining members (113, 114) each include an opening (134, 146) which each receive one said anchorage pin (135, 147) enabling anchorage of the assembly to vertebral bone.

7. An assembly according to claim 6 wherein the assembly is arranged so said retaining arms (132, 144) receive said retraction members (111, 112) in opposing relationship.

8. An assembly according to claim 7 wherein, each retraction member (111, 112) engages a corresponding formation (130, 142) on respective retaining arms (132, 144), allowing the retraction members (111, 112) to rotate about the retaining arms (132, 144) and slide therealong.

9. An assembly according to claim 8 wherein, the retaining arms (132, 144) are parallel.

10. An assembly according to claim 9 wherein, each said retaining arms (132, 144) have respective free ends.

11. An assembly according to claim 10 wherein, the formation (130, 142) on each said retraction member (111, 112) is a recess (166) which at least partially receives therein at least part of one said retaining arms (167) .

12. An assembly according to claim 11 wherein, the recess (166) engages the retaining arm (167) allowing the retraction member (160) to undergo said rotation and sliding movement relative to the retaining arm (167).

13. An assembly according to claim 12 wherein the first and second joining members (113, 114) each include a bifurcated portion defining opening (134) to allow penetration of an anchorage pin (135) for screw fixation of the assembly to bone.

14. An assembly according to claim 13 wherein, said retaining arms (132, 144) include a gripping region which co operates with the recess (166) in said retraction members (111, 112) and which facilitates a selective rotation position of the retractors (111, 112).

15. An assembly according to claim 14 wherein the retraction members (111, 112) are incrementally rotationally adjustable to increase or decrease soft tissue retraction.

16. An assembly according to claim 15 wherein said anchorage pins (135, 147) co -operate to allow distraction of vertebral bone segments when inserted in bone.

17. An assembly according to claim 16 wherein the assembly is capable of simultaneous distraction of bone and retraction of soft tissue.

18. An assembly according to claim 17 wherein each said retaining arm (132, 144) engages the anchor pins (135, 147) via said bifurcated portion.

19. An assembly according to claim 18 wherein, said anchorage pins (135, 147) are each retained by a sleeve (152,139).

20. An assembly for use during surgery in soft tissue retraction and bone distraction, wherein the assembly comprises; first and second joining members (113, 114) which each include a recess which receives an anchor pin (135) which engages vertebral bone; wherein at least one of said first or second joining members (113, 114) further comprises a retaining arm (132) which receives and detachably retains thereon a retraction member (111); and wherein the retraction member (111) has a first free end and a second end, the second end including a formation (130) which allows the retraction member (111) to be detachably retained by said retaining arm (132) thereby enabling said retraction member (111) to rotate about said retaining arm (132) for effecting soft tissue retraction and slide therealong during bone distraction through said anchor pins (135, 147).

21. An assembly according to claim 20 wherein the recess on each said first and second joining members (113, 114) comprises an opening (134) enabling screw fixation of the pins (135, 147) to allow anchorage of the assembly to the bone.

22. An assembly according to claim 21 comprising two retaining arms (132, 144) connected to said first and second joining members (113, 114) and which each receive retraction members (111, 112) in opposing relationship.

23. An assembly according to claim 22 wherein, each retraction member (111, 112) includes a formation (130, 142) which engages a corresponding formation on one said retaining arms (132, 144), thereby allowing the retraction member (111, 112) to rotate about the respective retaining arms (132, 144).

24. An assembly according to claim 23 wherein, the first and second retaining arms (132, 144) are parallel.

25. An assembly according to claim 24 further comprising an ancillary sleeve (153, 140) which engage anchor pins (135, 147) and receive a tool for enabling distraction of the vertebrae via the anchor pins (135, 147).

26. An assembly according to claim 25 wherein there are two anchor pins (135, 147) each receiving and retaining sleeves (152, 139) concentrically engaging said pins (135, 147); an auxiliary sleeve (140) which receives a tool to apply a distraction force to said pin (135) via said sleeve (140); the retaining arm (132) having a recess (134) capable of engaging one said pins (134) and which receives a first retraction member (111) via a co operating mating profile (130); a second retaining arm (144) having a first end including a recess capable of engaging the other of said pins (147) which releasably receives a second retraction member (112) via a mating profile (142); wherein said mating profiles (130, 142) each allow rotatable adjustment of each said retraction members (111, 112) to retract soft tissue margins during surgery.

27. An assembly according to claim 26 wherein, the retaining arms (132, 144) include spline formations (267) providing a mating engagement which allows longitudinal and rotational adjustment of said retraction members (111, 112).

28. An assembly according to claim 27 wherein, the spline formations (267) lock said retraction members (111, 112) in a selected rotational orientation.

29. An assembly according to claim 10 wherein said first and second joining members (113, 114) each include a bridging portion which spans at least the distance from the anchor pin (135, 147) it engages to one of the retaining arms (132, 144) wherein, each said retaining arms (132, 144) have a first end at the bridging portion and a second free end; the retentaining arms (132, 144) each receiving and retaining thereon a retraction member (111, 112) having a first free end and a second end, the second end including a formation (130, 142) which allows the retraction member (111, 112) to be detachably retained by one said retaining arm (132, 144) enabling said retraction members (111, 112) to rotate about said retaining arms (132, 144) and to slide therealong.

30. An assembly according to claim 29 wherein each bridging portion has an opening which receives one said anchorage pins (135, 147) for engaging bone thereby anchoring the assembly.

31. An assembly according to claim 30 wherein, each retraction member (111, 112) engages via said second end a corresponding formation on one said retaining arm (132, 1449

32. An assembly according to claim 31 wherein, each said retaining arms (132, 144) have a first end attached to said bridging portion and a second free end.

33. An assembly according to claim 32 wherein, the retaining arms (132, 144) are parallel and each disposed normally to the bridging portion.

## Patentansprüche

1. Anordnung, die fähig ist, um Weichgewebe zurückzuziehen und zur Distraktion von Knochen während einer Wirbelsäulenoperation, wobei die Anordnung aufweist:
eine Verankerung, umfassend:
ein erstes Verbindungselement (113), das mit einem ersten Verankerungsstift (135) kooperiert, der ein distales Ende (137) aufweist, das mit Wirbelknochen in Eingriff kommt und das das erste Verbindungselement (113) verankert; und
ein zweites Verbindungselement (114), das mit einem zweiten Verankerungsstift (147) kooperiert, der ein distales Ende (148) aufweist, das mit Wirbelknochen in Eingriff kommt und das das zweite Verbindungselement (114) verankert,
wobei eine relative Bewegung zwischen dem ersten und zweiten Verbindungselement (113, 114) während der Knochendistraktion durch die Verankerungsstifte (135, 147) gestattet ist,
und wobei das erste Verbindungselement (113) einen ersten Haltearm (132) umfasst, der ein erstes Rückzugselement (111) aufnimmt und hält, welches ein erstes freies Ende und ein zweites Ede hat, wobei das zweite Ende eine Formation (130) umfasst, die es dem ersten Rückzugselement (111) gestattet, an dem ersten Haltearm (132) gehalten zu werden und sich entlang des Haltearms (132) zu bewegen während der Distraktion von Knochen durch die Verankerungsstifte (135, 147).

2. Anordnung nach Anspruch 1, wobei das zweite Verbindungselement (114) einen zweiten Haltearm (144) aufweist, der ein zweites Rückzugselement (112) aufnimmt und hält, welches ein erstes freies Ende und ein zweites Ende hat, wobei das zweite Ende eine Formation (142) umfasst, die es dem zweiten Rückzugselement (112) gestattet, an dem Arm gehalten zu werden; wobei das erste und zweite Rückzugselement (111, 112) kooperieren, um Gewebe zurückzuziehen, während der erste und zweite Verankerungsstift (135, 147) kooperieren, um die Distraktion von Knochen zu bewirken.

3. Anordnung nach Anspruch 2, wobei jedes Rückzugselement (111,112) rotierbar ist um jeweilige Haltearme (132, 144).

4. Anordnung nach Anspruch 3, wobei die Rückzugselemente (111, 112) lösbar an den Haltearmen (132, 144) gehalten sind.

5. Anordnung nach Anspruch 4, wobei jeder Haltearm (132, 144) eine Rotationsachse für eines der Rückzugselemente (111, 112) bereitstellt.

6. Anordnung nach Anspruch 5, wobei das erste und zweite Verbindungselement (113, 114) jeweils eine Öffnung (134, 146) umfassen, die jeweils einen der Verankerungsstifte (135, 147) aufnehmen, die die Verankerung der Anordnung an Wirbelknochen ermöglichen.

7. Anordnung nach Anspruch 6, wobei die Anordnung ausgebildet ist, so dass die Haltearme (132, 144) die Rückzugselemente (111, 112) in entgegengesetzter Beziehung aufnehmen.

8. Anordnung nach Anspruch 7, wobei jedes Rückzugselement (111, 112) mit einer korrespondierenden Formation (130, 143) an jeweiligen Haltearmen (132, 144) in Eingriff steht, was den Rückzugselementen (111, 112) gestattet, um die Haltearme (132, 144) zu rotieren and daran entlang zu gleiten.

9. Anordnung nach Anspruch 8, wobei die Haltearme (132, 144) parallel sind.

10. Anordnung nach Anspruch 9, wobei jeder der Haltearme (132, 144) jeweils ein freies Ende hat.

11. Anordnung nach Anspruch 10, wobei die Formation (130, 142) an jedem Rückzugselement (111, 112) eine Ausnehmung (166) ist, die darin zumindest teilweise mindestens einen Teil der Haltearme (167) aufnimmt.

12. Anordnung nach Anspruch 11, wobei die Ausnehmung (166) mit dem Haltearm (167) in Eingriff steht, was es dem Rückzugselement (160) gestattet, der Rotation und Gleitbewegung relativ zu dem Haltearm (167) unterzogen zu werden.

13. Anordnung nach Anspruch 12, wobei die ersten und zweiten Verbindungselemente (113, 114) je einen gegabelten Bereich umfassen, der die Öffnung (134) definiert, um das Eindringen eines Verankerungsstifts (135) für eine Schraubbefestigung der Anordnung an Knochen zu gestatten.

14. Anordnung nach Anspruch 13, wobei die Haltearme (132, 144) eine Greifregion umfassen, die mit der Ausnehmung (166) in den Rückzugselementen (111, 112) kooperiert, und die eine selektive Rotationsposition der Rückzugselemente (111, 112) erleichtert.

15. Anordnung nach Anspruch 14, wobei die Rückzugselemente (111, 112) inkremental rotierbar einstellbar sind, um das Zurückziehen von Weichgewebe zu steigern oder zu verringern.

16. Anordnung nach Anspruch 15, wobei die Verankerungsstifte (135, 147) kooperieren, um die Distraktion von Wirbelknochensegmenten zu gestatten, wenn sie in Knochen eingesetzt sind.

17. Anordnung nach Anspruch 16, wobei die Anordnung zur simultanen Distraktion von Knochen und dem Zurückziehen von Weichgewebe in der Lage ist.

18. Anordnung nach Anspruch 17, wobei jeder Haltearm (132, 144) mit den Verankerungsstiften (135, 147) über den gabelförmigen Bereich in Eingriff steht.

19. Anordnung nach Anspruch 18, wobei die Verankerungsstifte (135, 147) jeweils durch eine Hülse (152, 139) gehalten sind.

20. Anordnung zur Verwendung während einer Operation im Zurückziehen von Weichgewebe und der Distraktion von Knochen, wobei die Anordnung aufweist:
erste und zweite Verbindungselemente (113, 114), die jeweils eine Ausnehmung aufweisen, die einen Verankerungsstift (135) aufnimmt, der mit Wirbelknochen in Eingriff kommt;
wobei mindestens eines der ersten oder zweiten Verbindungselemente (113, 114) weiterhin einen Haltearm (132) aufweist, der ein Rückzugselement (111) aufnimmt und lösbar daran hält;
und wobei das Rückzugselement (11) ein erstes freies Ende und ein zweites Ende hat, wobei das zweite Ende eine Formation (130) umfasst, die es dem Rückzugselement (111) gestattet, um lösbar von dem Haltearm (132) gehalten zu werden, um dadurch das Rückzugselement (811) zu befähigen, um den Haltearm (132) zu rotieren, um ein Zurückziehen von Weichgewebe und ein Gleiten daran entlang während der Distraktion von Knochen durch die Verankerungsstifte (135, 147) zu bewirken.

21. Anordnung nach Anspruch 20, wobei die Ausnehmung an jedem der ersten und zweiten Verbindungselemente (113, 114) eine Öffnung (134) aufweist, die eine Schraubbefestigung der Stifte (135, 147) ermöglicht, um eine Verankerung der Anordnung an Knochen zu gestatten.

22. Anordnung nach Anspruch 21, aufweisend zwei Haltearme (132, 144), die mit den ersten und zweiten Verbindungselementen (113, 114) verbunden sind, und die jeweils Rückzugselemente (111, 112) in entgegengesetzter Beziehung aufnehmen.

23. Anordnung nach Anspruch 22, wobei jedes Rückzugselement (111, 112) eine Formation (130, 142) umfasst, die mit einer korrespondierenden Formation an einem der Haltearme (132, 144) in Eingriff steht, und es dem Rückzugselement (111, 112) dadurch gestattet, um die jeweiligen Haltearme (132, 144) zu rotieren.

24. Anordnung nach Anspruch 23, wobei die ersten und zweiten Haltearme (132, 144) parallel sind.

25. Anordnung nach Anspruch 24, weiterhin aufweisend eine zusätzliche Hülse (153, 140), die mit den Verankerungsstiften (135, 147) in Eingriff steht und ein Werkzeug aufnimmt, um die Distraktion von Wirbelknochen über die Verankerungsstifte (135, 147) zu gestatten.

26. Anordnung nach Anspruch 25, wobei zwei Verankerungsstifte (135, 147) vorgesehen sind, die jeweils Hülsen (152, 139) aufnehmen und halten, die mit den Stiften (135, 147) konzentrisch in Eingriff stehen;
eine Hilfshülse (140), die ein Werkzeug aufnimmt, um über die Hülse (140) eine Distraktionskraft auf den Stift (135) auszuüben;
wobei der Haltearm (132) eine Ausnehmung (134) hat, die in der Lage ist, mit einem der Stifte (134) in Eingriff zu kommen und die ein erstes Rückzugselement (111) über ein kooperierendes Verpaarungsprofil (130) aufnimmt;
wobei ein zweiter Haltearm (140) ein erstes Ende hat, das eine Ausnehmung umfasst, die in der Lage ist, mit dem anderen der Stifte (147) in Eingriff zu kommen, die lösbar ein zweites Rückzugselement (112) über ein Verpaarungsprofil (142) aufnimmt,
wobei die Verpaarungsprofile (130, 142) jeweils eine rotierbare Einstellung von jedem der Rückzugselemente (111,112) gestatten, um Weichgeweberänder während der Operation zurückzuziehen.

27. Anordnung nach Anspruch 26, wobei die Haltearme (132, 144) Spline-Formationen (267) umfassen, die einen Verpaarungs-Eingriff bereitstellen, welcher eine longitudinale und drehende Einstellung der Rückzugselemente (111, 112) gestattet.

28. Anordnung nach Anspruch 27, wobei die Spline-Formationen (267) die Rückzugselemente (111, 112) in einer ausgewählten drehenden Orientierung verriegeln.

29. Anordnung nach Anspruch 10, wobei die ersten und zweiten Verbindungselemente (113, 114) jeweils einen Überbrückungsbereich umfassen, der mindestens den Abstand von dem Verankerungsstift (135, 147) überspannt, er steht in Eingriff mit einem der Haltearme (132, 144), wobei jeder der Haltearme (132, 144) ein erstes Ende an dem Überbrückungsbereich und ein zweites Ende hat;
wobei die Haltearme (132, 144) jeweils ein Rückzugselement (111, 112) aufnehmen und daran halten, die ein erste freies Ende und ein zweites Ende haben, wobei das zweite Ende eine Formation (130, 142) umfasst, die es dem Rückzugselement (111, 112) gestattet, lösbar durch einen der Haltearme (132, 144) gehalten zu werden, wodurch die Rückzugselemente (111, 112) in die Lage versetzt werden, um um die Haltearme (132, 144) zu rotieren und daran entlang zu gleiten.

30. Anordnung nach Anspruch 29, wobei jeder Überbrückungsbereich eine Öffnung hat, die einen der Verankerungsstifte (135, 147) aufnimmt, zum in Eingriff zu kommen mit Knochen und dadurch Verankern der Anordnung.

31. Anordnung nach Anspruch 30, wobei jedes Rückzugselement (111, 112) über das zweite Ende in Eingriff kommt mit einer korrespondierenden Formation an einem der Haltearme (132, 144).

32. Anordnung nach Anspruch 31, wobei jeder der Haltearme (132, 144) ein erstes Ende hat, das an dem Überbrückungsbereich angeordnet ist, und ein zweites freies Ende.

33. Anordnung nach Anspruch 32, wobei die Haltearme (132, 144) parallel sind und jeweils normal zu dem Überbrückungsbereich angeordnet sind.

## Revendications

1. Ensemble capable d'une rétractation de tissu mou et d'une distraction osseuse pendant une chirurgie rachidienne, l'ensemble comprenant :
un ancrage comprenant :
un premier élément de liaison (113) qui coopère avec une première broche d'ancrage (135) ayant une extrémité distale (137) qui engage un os vertébral et qui ancre le premier élément de liaison (113) ; et
un second élément de liaison (114) qui coopère avec une seconde broche d'ancrage (147) ayant une extrémité distale (148) qui engage un os vertébral et qui ancre le second élément de liaison (114) ;
un mouvement relatif entre les premier et second éléments de liaison (113, 114) étant autorisé pendant une distraction osseuse par lesdites broches d'ancrage (135, 147) ; et
le premier élément de liaison (113) comprenant un bras de retenue (132) qui reçoit et retient un premier élément de rétractation (111) ayant une première extrémité libre et une seconde extrémité, la seconde extrémité comprenant une formation (130) qui permet au premier élément de rétractation (111) d'être retenu sur le bras de retenue (132) et de se déplacer le long du bras de retenue (132) pendant une distraction osseuse par les broches d'ancrage (135, 147).

2. Ensemble selon la revendication 1, dans lequel le second élément de liaison (114) comprend un second bras de retenue (144) qui reçoit et retient un second élément de rétractation (112) ayant une première extrémité libre et une seconde extrémité, la seconde extrémité comprenant une formation (142) qui permet au second élément de rétractation (112) d'être retenu sur le bras ; lesdits premier et second éléments de rétractation (111, 112) coopérant pour rétracter du tissu mou tandis que lesdites première et seconde broches d'ancrage (135, 147) coopèrent pour effectuer ladite distraction osseuse.

3. Ensemble selon la revendication 2, dans lequel chaque élément de rétractation (111, 112) est apte à tourner autour de bras de retenue respectifs (132, 144).

4. Ensemble selon la revendication 3, dans lequel les éléments de rétractation (111, 112) sont retenus de manière détachable sur les bras de retenue (132, 144).

5. Ensemble selon la revendication 4, dans lequel chaque bras de retenue (132, 144) fournit un axe de rotation pour l'un desdits éléments de rétractation (111, 112) .

6. Ensemble selon la revendication 5, dans lequel les premier et second éléments de liaison (113, 114) comprennent chacun une ouverture (134, 146) qui reçoit une broche d'ancrage précitée (135, 147) permettant un ancrage de l'ensemble à un os vertébral.

7. Ensemble selon la revendication 6, l'ensemble étant agencé de telle sorte que lesdits bras de retenue (132, 144) reçoivent lesdits éléments de rétractation (111, 112) en relation opposée.

8. Ensemble selon la revendication 7, dans lequel chaque élément de rétractation (111, 112) engage une formation correspondante (130, 142) sur des bras de retenue respectifs (132, 144), permettant aux éléments de rétractation (111, 112) de tourner autour des bras de retenue (132, 144) et de coulisser le long de ceux-ci.

9. Ensemble selon la revendication 8, dans lequel les bras de retenue (132, 144) sont parallèles.

10. Ensemble selon la revendication 9, dans lequel chacun desdits bras de retenue (132, 144) a des extrémités libres respectives.

11. Ensemble selon la revendication 10, dans lequel la formation (130, 142) sur chaque élément de rétractation précité (111, 112) est un évidement (166) qui reçoit au moins partiellement, à l'intérieur de celui-ci, au moins une partie de l'un desdits bras de retenue (167).

12. Ensemble selon la revendication 11, dans lequel l'évidement (166) engage le bras de retenue (167) permettant à l'élément de rétractation (160) de subir ladite rotation et ledit mouvement de coulissement par rapport au bras de retenue (167).

13. Ensemble selon la revendication 12, dans lequel les premier et second éléments de liaison (113, 114) comprennent chacun une ouverture (134) définissant une partie bifurquée, pour permettre une pénétration d'une broche d'ancrage (135) pour fixation par vis de l'ensemble à un os.

14. Ensemble selon la revendication 13, dans lequel lesdits bras de retenue (132, 144) comprennent une région de saisie qui coopère avec l'évidement (166) dans lesdits éléments de rétractation (111, 112) et qui facilite une position de rotation sélective des éléments de rétractation (111, 112).

15. Ensemble selon la revendication 14, dans lequel les éléments de rétractation (111, 112) sont ajustables en rotation de manière incrémentale pour accroître ou diminuer une rétractation de tissu mou.

16. Ensemble selon la revendication 15, dans lequel lesdites broche d'ancrage (135, 147) coopèrent pour permettre une distraction de segments osseux vertébraux lorsqu'elles sont introduites dans un os.

17. Ensemble selon la revendication 16, l'ensemble étant capable d'une distraction osseuse et d'une rétractation de tissu mou simultanées.

18. Ensemble selon la revendication 17, dans lequel chaque bras de retenue (132, 144) engage les broches d'ancrage (135, 147) par l'intermédiaire de ladite partie bifurquée.

19. Ensemble selon la revendication 18, dans lequel lesdites broches d'ancrage (135, 147) sont chacune retenues par un manchon (152, 139).

20. Ensemble destiné à être utilisé pendant une chirurgie de rétractation de tissu mou et de distraction osseuse, l'ensemble comprenant :
des premier et second éléments de liaison (113, 114) qui comprennent chacun un évidement qui reçoit une broche d'ancrage (135) qui engage un os vertébral ;
au moins un parmi lesdits premier et second éléments de liaison (113, 114) comprenant en outre un bras de retenue (132) qui reçoit et retient de manière détachable, sur celui-ci, un élément de rétractation (111) ; et
l'élément de rétractation (111) ayant une première extrémité libre et une seconde extrémité, la seconde extrémité comprenant une formation (130) qui permet à l'élément de rétractation (111) d'être retenu de manière détachable par ledit bras de retenue (132), permettant ainsi audit élément de rétractation (111) de tourner autour dudit bras de retenue (132) pour réaliser une rétractation de tissu mou et de coulisser le long de celui-ci pendant une distraction osseuse par lesdites broches d'ancrage (135, 147).

21. Ensemble selon la revendication 20, dans lequel l'évidement sur chacun desdits premier et second éléments de liaison (113, 114) comprend une ouverture (134) permettant une fixation par vis des broches (135, 147) pour permettre un ancrage de l'ensemble à l'os.

22. Ensemble selon la revendication 21, comprenant deux bras de retenue (132, 144) reliés auxdits premier et second éléments de liaison (113, 114) et qui reçoivent chacun des éléments de rétractation (111, 112) en relation opposée.

23. Ensemble selon la revendication 22, dans lequel chaque élément de rétractation (111, 112) comprend une formation (130, 142) qui engage une formation correspondante sur l'un desdits bras de retenue (132, 144), permettant ainsi à l'élément de rétractation (111, 112) de tourner autour des bras de retenue respectifs (132, 144).

24. Ensemble selon la revendication 23, dans lequel les premier et second bras de retenue (132, 144) sont parallèles.

25. Ensemble selon la revendication 24, comprenant en outre un manchon auxiliaire (153, 140) qui engage des broches d'ancrage (135, 147) et reçoit un outil pour permettre une distraction des vertèbres par les broches d'ancrage (135, 147).

26. Ensemble selon la revendication 25, dans lequel il y a deux broches d'ancrage (135, 147) recevant et retenant chacune des manchons (152, 139) engageant lesdites broches (135, 147) de manière concentrique ;
un manchon auxiliaire (140) qui reçoit un outil pour appliquer une force de distraction à ladite broche (135) par l'intermédiaire dudit manchon (140) ;
le bras de retenue (132) ayant un évidement (134) capable d'engager l'une desdites broches (134) et qui reçoit un premier élément de rétractation (111) par l'intermédiaire d'un profil d'accouplement par coopération (130) ;
un second bras de retenue (144) ayant une première extrémité comprenant un évidement capable d'engager l'autre desdites broches (147) qui reçoit un second élément de rétractation (112) de manière libérable par l'intermédiaire d'un profil d'accouplement (142) ;
lesdits profils d'accouplement (130, 142) permettant chacun un ajustement en rotation de chacun desdits éléments de rétractation (111, 112) pour rétracter des bords de tissu mou pendant une chirurgie.

27. Ensemble selon la revendication 26, dans lequel les bras de retenue (132, 144) comprennent des formations de cannelure (267) fournissant un engagement par accouplement qui permet un ajustement longitudinal et en rotation desdits éléments de rétractation (111, 112).

28. Ensemble selon la revendication 27, dans lequel les formations de cannelure (267) verrouillent lesdits éléments de rétractation (111, 112) dans une orientation de rotation sélectionnée.

29. Ensemble selon la revendication 10, dans lequel lesdits premier et second éléments de liaison (113, 114) comprennent chacun une partie pont qui couvre au moins la distance allant de la broche d'ancrage (135, 147) qu'elle engage jusqu'à l'un des bras de retenue (132, 144), chacun desdits bras de retenue (132, 144) ayant une première extrémité au niveau de la partie pont et une seconde extrémité libre ;
les bras de retenue (132, 144) recevant et retenant chacun, sur ceux-ci, un élément de rétractation (111, 112) ayant une première extrémité libre et une seconde extrémité, la seconde extrémité comprenant une formation (130, 142) qui permet à l'élément de rétractation (111, 112) d'être retenu de manière détachable par ledit bras de retenue (132, 144), permettant auxdits élément de rétractation (111, 112) de tourner autour desdits bras de retenue (132, 144) et de coulisser le long de ceux-ci.

30. Ensemble selon la revendication 29, dans lequel chaque partie pont a une ouverture qui reçoit l'une desdites broches d'ancrage (135, 147) pour engager un os, ancrant ainsi l'ensemble.

31. Ensemble selon la revendication 30, dans lequel chaque élément de rétractation (111, 112) engage, par l'intermédiaire de ladite seconde extrémité, une formation correspondante sur ledit bras de retenue (132, 144).

32. Ensemble selon la revendication 31, dans lequel chacun desdits bras de retenue (132, 144) a une première extrémité fixée à ladite partie pont et une seconde extrémité libre.

33. Ensemble selon la revendication 32, dans lequel les bras de retenue (132, 144) sont parallèles et sont chacun disposés perpendiculairement à la partie pont.
